(19) Europäisches Patentamt
European Patent Office
Office européen des brevets

(11) Publication number: **0 443 498 A1**

# (12) EUROPEAN PATENT APPLICATION

(21) Application number: **91102291.1**

(22) Date of filing: **18.02.91**

(51) Int. Cl.5: **C07D 401/04**, C07D 207/10, C07D 207/12, C07D 207/14, C07D 309/12, C07C 31/34, C07C 271/16, C07C 309/42, A61K 31/47

(30) Priority: **19.02.90 JP 37819/90**

(43) Date of publication of application: **28.08.91 Bulletin 91/35**

(84) Designated Contracting States: **BE CH DE ES FR GB IT LI NL SE**

(71) Applicant: **KYORIN PHARMACEUTICAL CO., LTD.**
**No. 5, Kanda Surugadai 2-chome**
**Chiyoda-ku Tokyo(JP)**

(72) Inventor: **Asahina, Yoshikazu**
**No. 5982-1, Tomonuma, Nogi-machi**
**Shimotsuga-gun, Tochigi-ken(JP)**
Inventor: **Fukuda, Yasumichi**
**No. 6096 Tomonuma, Nogi-machi**
**Shimotsuga-gun, Tochigi-ken(JP)**
Inventor: **Fukuda, Hideyuki**
**No. 387, Touya-machi**
**Utsunomiya-shi, Tochigi-ken(JP)**

(74) Representative: **ter Meer, Nicolaus, Dipl.-Chem.Dr.**
**Patentanwälte ter Meer, Müller, Steinmeister**
**Mauerkircherstrasse 45**
**W-8000 München 80(DE)**

(54) **Optically active 8-methoxyquinolonecarb-oxylic acid derivatives, their preparative processes and their intermediates.**

(57) Optically active 8-methoxyquinolonecarboxylic acids represented by the formula (I)

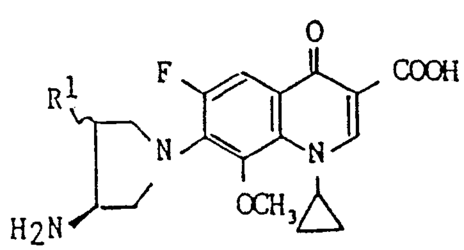

(I)

wherein $R^1$ is lower alkyl have been found to possess potent antibacterial activity against both Gram-negative and Gram-positive bacteria. The compounds may be synthesized from novel optically active intermediates.

EP 0 443 498 A1

# OPTICALLY ACTIVE 8-METHOXYQUINOLONECARBOXYLIC ACID DERIVATIVES, THEIR PREPARATIVE PROCESSES AND THEIR INTERMEDIATES

The present invention relates to novel quinolonecarboxylic acid derivatives, which have excellent antibacterial properties, their preparative processes, and antibacterial agents having these compounds as effective ingredients.

## Discussion of the Prior Art

The quinolonecarboxylic acid-based antibacterial agents such as norfloxacin, ofloxacin and ciprofloxacin, now universally used clinically, exhibit potent activity against Gram-negative bacteria including Pseudomonas aeruginosa, but the antibacterial potency against Gram-positive bacteria is considerably inferior to that against Gram-negative bacteria. They leave behind therefore a clinical problem of increased frequencies in the isolation of Gram-positive bacteria. It has become clear that 7-(3-amino-4-methyl-l-pyrrolidinyl)-1-cyclopropyl-6-fluoro-1,4-dihydro-8-methoxy-4-oxo-3-quinolinecarboxylic acid (Japanese Unexamined Patent Publication No. Sho 62-252772; U.S. Patent 4,980,470) prepared first by the present applicant exhibits very potent activity not only against Gram-negative bacteria but also against Gram-positive bacteria. For this compound, four types of optical isomers exist based on the two asymmetric carbon atoms on 3-amino-4-methylpyrrolidine ring being a substituent at 7-position, but the biological activities such as antibacterial potency of each isomer have not been made clear.

## SUMMARY OF THE INVENTION

The inventors synthesized these four types of optical isomers and studied extensively about their biological activities. As a result, it has been made clear that, to the inventors surprise, 7-[3(S)-amino-4(S)-methyl-l-pyrrolidinyl]-and 7-[3 (S)-amino-4(R)-methyl-1-pyrrolidinyl]-1-cyclopropyl-6-fluoro-1,4-dihydro-8-methoxy-4-oxo-3-quinolinecarboxylic acids, amino group at 3-position on pyrrolidine ring at 7-position being S-configuration, exhibit two to three times stronger in-vitro antibacterial potency over compounds, amino group at 3-position being R-configuration, and further that preventive effect on infection against Staphylococcus aureus being a Gram-positive bacterium is about six times higher, leading to the completion of the invention.

Since the compounds of the invention exhibit equal or more potent activity against Gram-negative bacteria compared with ciprofloxacin and simultaneously they show very potent antibacterial effect about twenty times higher against Gram-positive bacteria, they are very useful as medicinal drugs for human beings and livestock and further as antibacterial agents for fish, shellfish and plants.

Based on the above findings, the present invention provides optically active compounds represented by the formula [I]

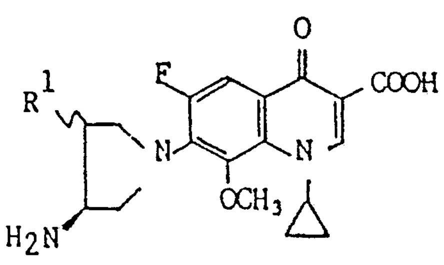

[I]

wherein $R^1$ is lower alkyl, the absolute configuration being S-or R-configuration, hydrates thereof or pharmaceutically acceptable salts thereof.

In a further embodiment, the present invention provides a process for the preparation of an optically active compounds represented by the above-noted formula [I] comprising:

condensing a compound of the formula [II]

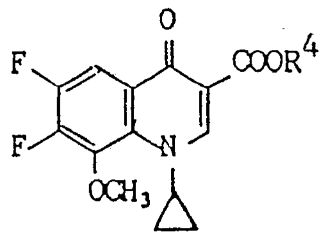

[II]

wherein $R^4$ is a hydrogen or lower alkyl with a compound of the formula [III]

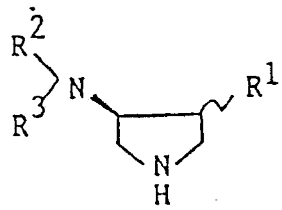

[III]

wherein $R^1$ is as defined above, and $R^2$ and $R^3$ each independently represent hydrogen or a protective group for the amino, or $R^2$ and $R^3$ together form a protective group for the amino; and

when $R^2$ or $R^3$ is a protective group, or when $R^2$ and $R^3$ together form a protective group eliminating the protective groups; and

when $R^4$ is lower alkyl, hydrolyzing the carboxylic acid ester to convert $R^4$ to H.

In a still further embodiment of the invention, the present invention provides an antibacterial agent comprising an optically active, antibacterially effective amount of an 8-methoxy-quinolonecarboxylic acid represented by the formula [I], above, a hydrate thereof, or a pharmaceutically acceptable salt thereof; and a pharmaceutically acceptable diluent.

In another embodiment of the invention, the present invention provides a method of treating a bacterial infection in a patient comprising administering to said patient an optically active antibacterially effective amount of an 8-methoxyquinolonecarboxylic acid represented by the formula [I], above, a hydrate thereof or a pharmaceutically acceptable salt thereof.

In another embodiment of the invention, the present invention provides an optically active pyrrolidine represented by the formula [IV]

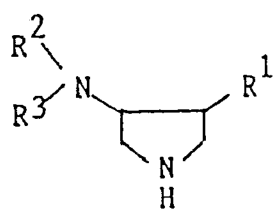

wherein $R^1$ is lower alkyl, and $R^2$ and $R^3$ each independently represent hydrogen or a protective group for the amino, or $R^2$ and $R^3$ together form a protective group for the amino; wherein the substituent at the 3-position is in the S- or R-configuration and the substituent at the 4-position is in the S- or R-configuration; and processes for the preparation thereof. In still another embodiment, the present invention provides an optically active pyrrolidine represented by the formula [V]

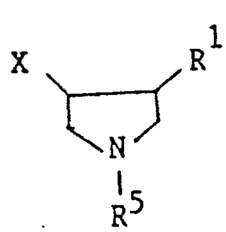

[V]

wherein $R^5$ is benzyl, diphenylmethyl, trityl, p-methoxybenzyl, p-methoxyphenyl or 1-phenethyl, $R^1$ is lower alkyl, and X is amino, protected amino, hydroxyl, protected hydroxyl, p-toluenesulfonyloxy, methanesulfonyloxy, azido or halogen; and wherein the substituent at the 3-position is in the S- or R-configuration and the substituent at the 4-position is in the S- or R-configuration. In yet another embodiment, the present invention provides an optically active butane represented by the formula [VI]

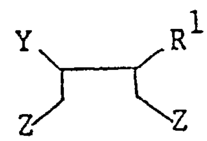

[VI]

wherein Y is a protected amino, a hydroxyl, a protected hydroxyl or halogen,
Z is hydroxyl, p-toluenesulfonyloxy, methanesulfonyloxy or halogen, and
$R^1$ is lower alkyl; and wherein the substituent at the 2-position is in S- or R-configuration and the substituent at the 3-position is in S- or R-configuration.

## DETAILED DESCRIPTION OF THE INVENTION

The following reaction scheme is illustrative of the preparation of the optically active 8-methoxyquinolonecarboxylic acids of the present invention:

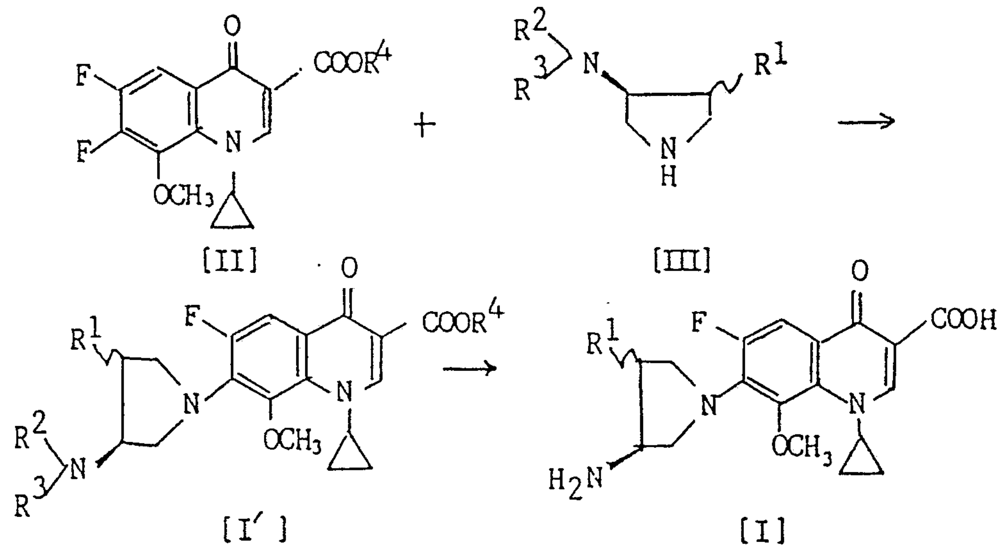

wherein $R^4$ indicates hydrogen or lower alkyl (e.g., 1 to 6 carbon atoms), $R^1$ indicates methyl, the absolute configuration being S-or R-configuration, and $R^2$ and $R^3$ each indicate independently hydrogen atom or protective group or indicate cooperatively a protective group.

Namely, by allowing compounds represented by the formula [II] to react with amines represented by the formula [III], the compounds of the invention represented by the formula [I] can be obtained. However, when either or both of $R^2$ and $R^3$ is(are) protective group(s) for amino group, for example, lower acyl groups such as acetyl and propionyl group or lower alkoxycarbonyl groups such as methoxycarbonyl, ethoxycarbonyl and t-butoxycarbonyl (Boc) group, or $R^2$ and $R^3$ form a phthaloyl group in the formula [III], the compounds of the invention can be obtained by removing protective group(s) according to methods well-known in the art. Also, in the case of compounds, $R^4$ being a lower alkyl group in the formula [III], the reaction resultants with compounds represented by the formula [III] are hydrolyzed according to methods well known in the art, and esters are converted to carboxylic acids to obtain the compounds of the invention.

The reaction between compounds represented by the formula [II] and compounds represented by the formula [III] can be carried out without solvent or in solvents such as water, alcohols, acetonitrile, dimethylformamide (DMF), dimethyl sulfoxide (DMSO), hexamethylphosphoric amide, pyridine and picoline. The reaction temperature is selected within a range from room temperature to 200° C, preferably from room

temperature to 160 ° C.

In more detail, it is suitable to allow 1 mol of compounds represented by the formula [II] to react with 1 to 5 mols of compounds represented by the formula [III] for 1 to several hours at room temperature to 160 ° C in 2 to 10 volumes of said solvent, based on the volume of the reactants. At this time, the use of deacidifying agents such as triethylamine, diazabicyclo base and potassium carbonate is also preferable. Moreover, compounds, $R^4$ being a lower alkyl group and/or $R^2$ and $R^3$ being protective groups for amine in the formula [I'], can be hydrolyzed according to usual methods. Such hydrolysis can be easily carried out at from room temperature to the boiling point of the solvent in water, water-alcohol mixed liquors, water-acetic acid mixed liquor, etc. by using alkaline bases such as caustic potash or acids such as sulfuric acid.

Moreover, compounds shown by a formula [XIV], the absolute configuration of amino group being R, can also be synthesized by allowing compounds shown by the formula [II] to react with compounds shown by formula [XIII] in the same way as above.

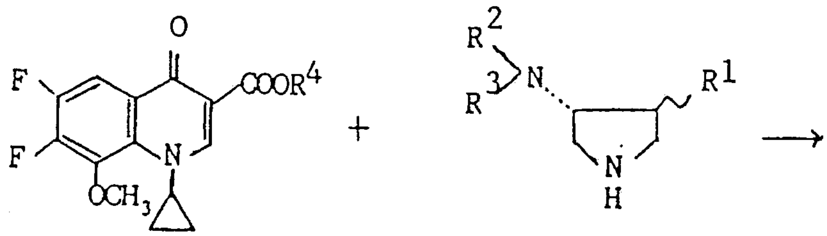

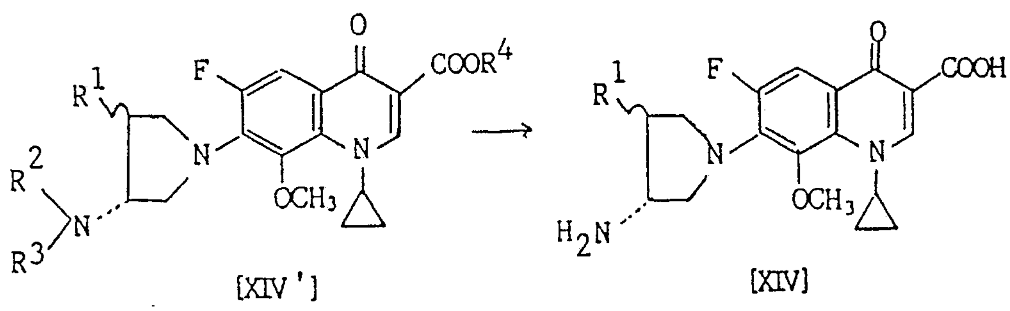

[XIV ']

[XIV]

wherein $R^1$, $R^2$, $R^3$ and $R^4$ are same as above.

Furthermore, the optically active pyrrolidine derivatives represented by the general formula [IV] being starting materials of the compounds of the invention are also novel compounds and can be synthesized through following routes using, for example, L- or D-aspartic acid or L- or D-malic acid as a raw material.

Route A

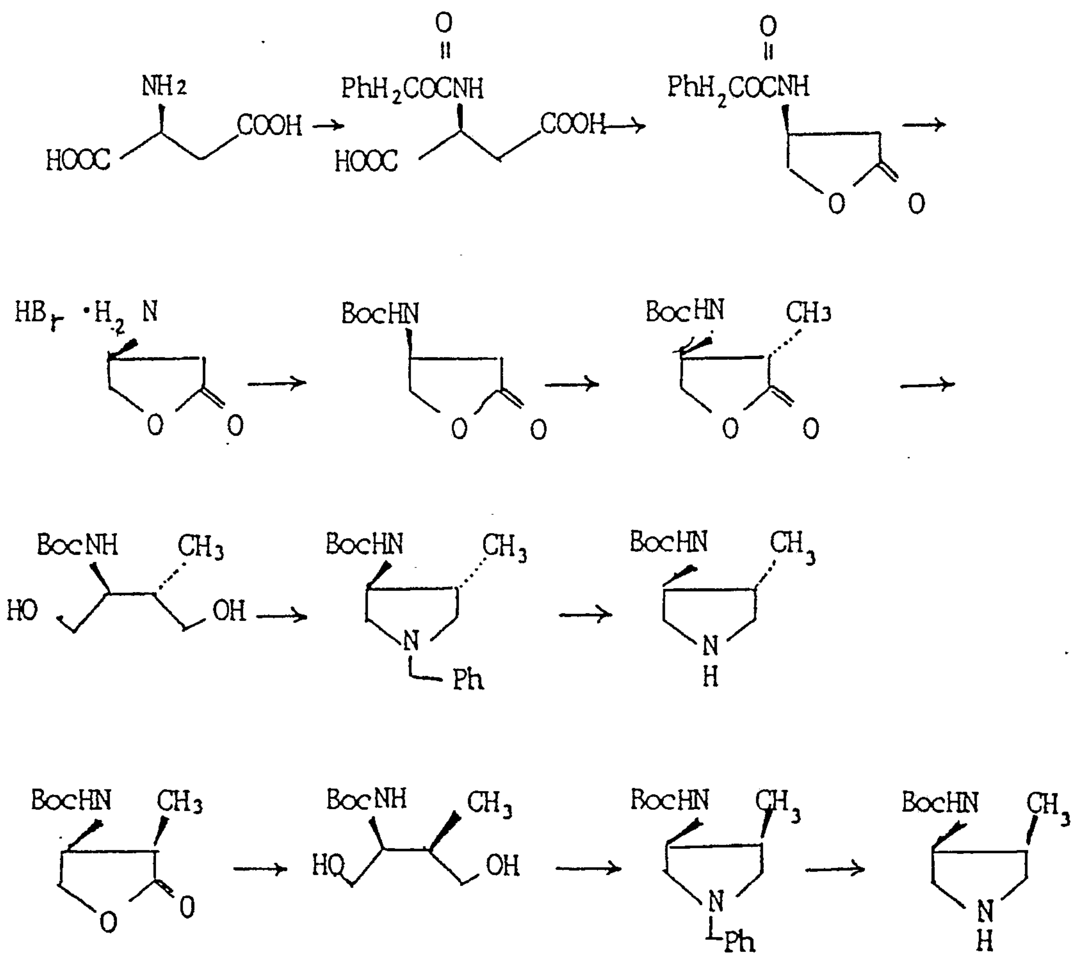

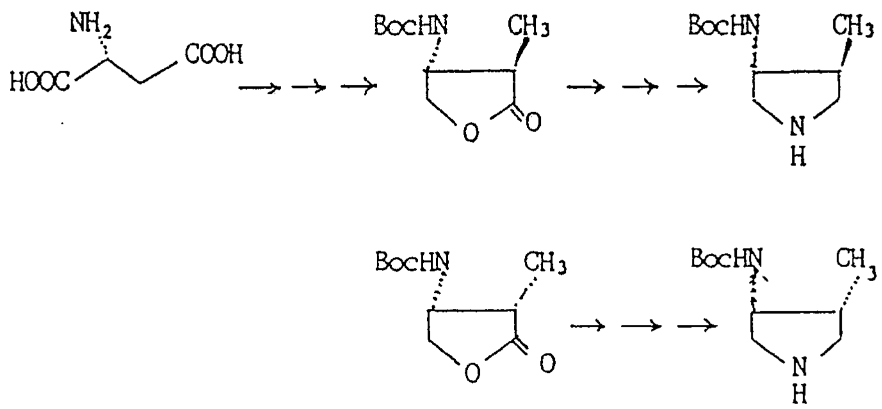

Route B

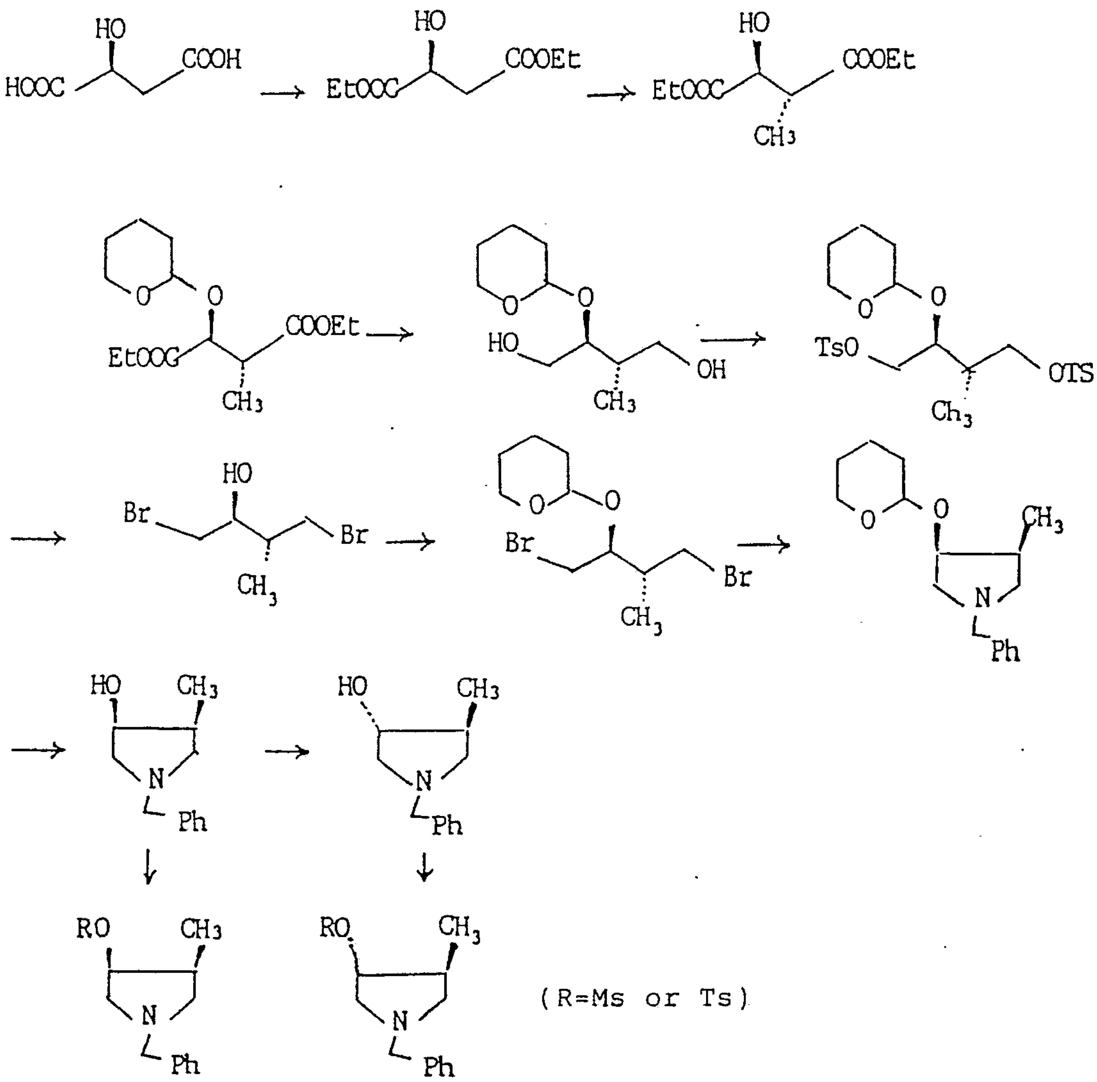

(R=Ms or Ts)

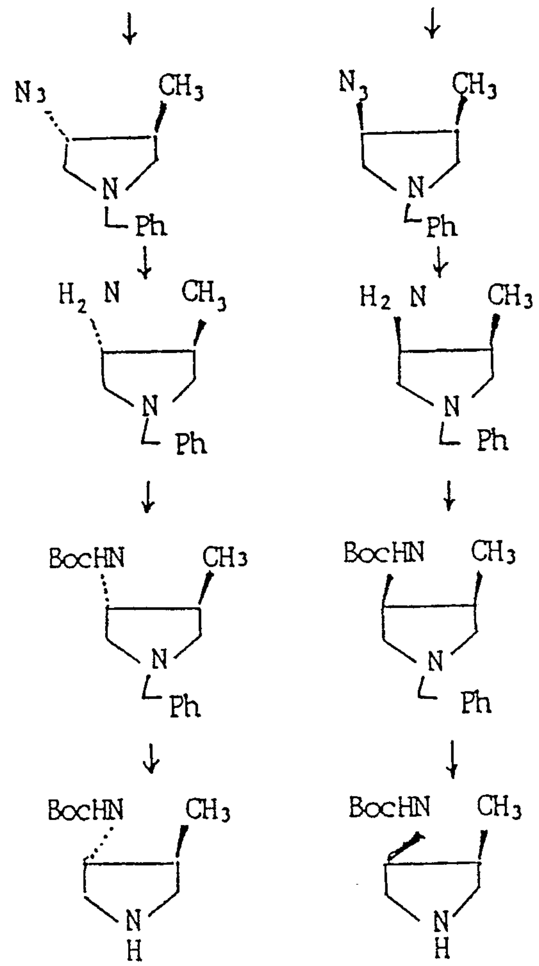

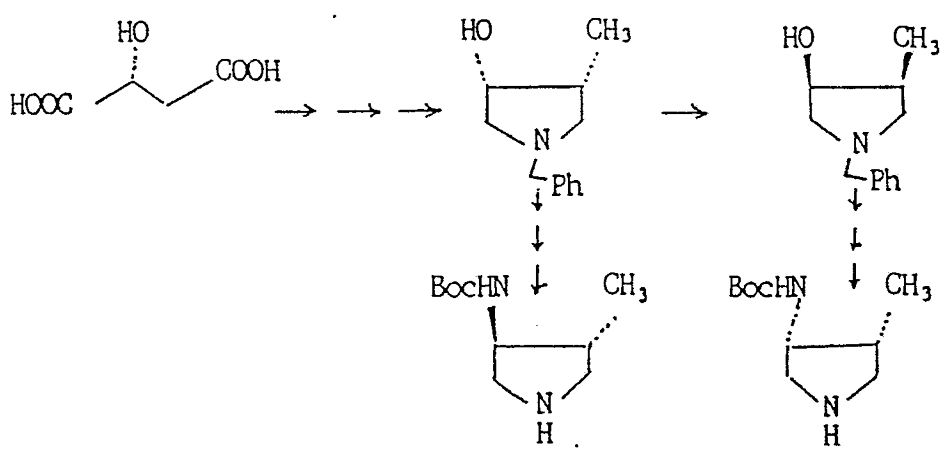

Next, the compounds represented by the formula [I] can be converted to their pharmaceutically acceptable salts according to usual methods, if desirable. As the salts, for example, salts with inorganic acids such as hydrochloric acid, sulfuric acid and phosphoric acid, salts with organic acids such as methanesulfonic acid, lactic acid, oxalic acid and acetic acid, or metal salts with sodium, potassium, magnesium, aluminum, cerium, chromium, cobalt, copper, iron, zinc, platinum, silver, etc. can be mentioned. Moreover, the compounds of the invention not only exhibit very good absorptivity when administered orally to animals, but also do not show any particularly problematic toxic effect in oral and parenteral administration. They are very useful therefore as medicinal drugs for human beings, animals, fish and shellfish and further as agricultural chemicals for plants.

Furthermore, when administering the compounds of the invention to human beings or animals and plants, forms and routes well-known pharmaceutically are applied. For example, they are used orally or parenterally through powders, tablets, capsules, ointments, injections, syrups, liquids, eye drops, suppositories, etc. As will be appreciated, the administration of the present compounds can be effected in the same manner and with similar dosage levels to the known quinolonecarboxylic acid-based antibacterial agents, i.e. norfloxacin, ofloxacin and ciprofloxacin, taking into account the relative potency of the present compounds with respect to Gram-negative and Gram-positive bacteria.

In the following, the compounds of the invention and their preparative processes will be illustrated in detail, however, such illustrations are not limiting on the scope of the invention.

Referential Example 1

Synthesis of 3(S)-[(t-butoxycarbonyl)amino]-γ-butyrolactone

Into 800 ml of water were dissolved 66.6 g of L-aspartic acid and 90.9 g of potassium carbonate, and 46.4 ml of carbobenzoxy chloride were added dropwise. After stirring the mixture for 2 hours at room temperature, 40.0 g of potassium carbonate were added and 46.4 ml of carbobenzoxy chloride were added dropwise, which was further stirred for 4 hours at room temperature. After washing the reaction liquor with ether, the pH was brought to 2 with concentrated hydrochloric acid, which was extracted with ethyl acetate. The ethyl acetate layer was separated, dried over anhydrous Glauber's salt, and then concentrated. The residue was crystallized with ether-n-hexane and the crystals deposited were collected by filtration, and then washed with n-hexane to obtain 108.6 g of 2(S)-[(benzyloxycarbonyl)amino]butanoic diacid.

To 100 ml of acetic anhydride were added 106.9 g of 2(S)-[(benzyloxycarbonyl)amino]butanoic diacid, and the mixture was stirred for 2 hours at room temperature. The reaction liquor was concentrated below 40 °C and the residue was washed with ether-n-hexane (5:1). 600 ml of anhydrous tetrahydrofuran (THF) dissolved these crystals and the solution was added dropwise to a solution of 500 ml of anhydrous tetrahydrofuran containing 14.4 g of dissolved sodium borohydride at 0 °C, which was then stirred for 1 hr at room temperature. To the reaction liquor was added 6N hydrochloric acid to bring the pH to 2, and the solution was concentrated to about 1/4 by volume and extracted with ether after added water. The ether layer was separated, washed with water and then washed with saturated saline solution, and then concentrated. To the residue were added 200 ml of benzene and 0.4 g of p-toluenesulfonic acid, and the mixture was refluxed for 4 hours under heat. The reaction liquor was concentrated and the residue was crystallized with n-hexane-ether to obtain 82.5 g of 3(S)-[(benzyloxycarbonyl)amino]-γ-butyrolactone as colorless prismatic crystals.

Into 50 ml of acetic acid were dissolved 11.5 g of 3(S)-[(benzyloxycarbonyl)amino]-γ-butyrolactone, and the solution was stirred for 4 hours at room temperature while passing hydrogen bromide, generated from 5 ml of bromine and 40 g of tetralin, therethrough. Ether was added to the reaction liquor, which was cooled. The crystals deposited were collected by filtration and washed with ether and then with small amount of acetone to obtain 8.0 g of 3(S)-amino-γ-butyrolactone•hydrobromide.

Into 40 ml of anhydrous methylene chloride were suspended 5.46 g of 3 (S)-amino-γ-butyrolactone•hydrobromide, and, after dropwise addition of a solution of 20 ml of anhydrous methylene chloride, 4.18 ml of triethylamine and 6.55 g of di-t-butyl dicarbonate under cooling with ice, the mixture was stirred for 4 hours at room temperature. The reaction liquor was washed with water and then with saturated saline solution, dried over magnesium sulfate, and then concentrated. The residual crystals were washed with ether to obtain 5.88 g of aimed product as colorless needle-like crystals.

Melting point: 114 - 116 °C

Specific rotation: $[\alpha]_D^{20}$ = -61.3° (c = 1.00, chloroform)

Elemental analysis: $C_9H_{15}NO_4$

| | | | |
|---|---|---|---|
| Calculated | C: 53.72 | H: 7.51 | N: 6.96 |
| Observed | C: 53.69 | H: 7.42 | N: 6.89 |

Referential Example 2

Synthesis of 3(R)-[(t-butoxycarbonyl)-amino]-γ-butyrolactone

The reaction was performed similarly to Referential Example 1 starting from 50.0 g of D-aspartic acid to obtain 9.55 g of aimed product.

Referential Example 3

Synthesis of 2(S)-[(2-tetrahydropyranyl)oxy]-3(S)-n-ethyl-1,4-butanediol

Into 120 ml of ethanol were dissolved 30.0 g of L-malic acid, and, after addition of a catalytic amount of thionyl chloride, the mixture was refluxed for 1 hour under heat. The reaction liquor was concentrated and the residue was purified by distillation under reduced pressure to obtain 31.9 g of diethyl L-malate having a boiling point of 110-120 °C (3 mmHg) and being colorless oil.

While cooling a solution of 200 ml of anhydrous THF containing 32.5 ml of dissolved diisopropylamine in a salt-ice bath, 132.0 ml of n-butyllithium (1.6 M hexane solution) were added dropwise below 0 °C. To this solution cooled in a dry ice-acetone bath was added dropwise a solution of 20 ml of anhydrous tetrahydrofuran, 20.0 g of diethyl L-malate and 18.8 g of anhydrous hexamethylphosphoric triamide. After stirred for 40 minutes at -20 °C, 9.9 ml of methyl iodide were added dropwise while cooling again in a dry ice-acetone bath. After stirring the reaction liquor for 30 minutes at room temperature, 1 M aqueous solution of citric acid was added to the reaction liquor, which was extracted with ethyl acetate. Ethyl acetate layer was separated, washed with saturated saline solution, dried over anhydrous Glauber's salt, and then concentrated. The residue was purified through silica gel column (elution solvent, n-hexane:ethyl acetate = 4:1) to obtain 17.8 g of diethyl 2(S)-hydroxy-3(R)-methyl-1,4-butanoate as yellow oil.

Into 80 ml of anhydrous ether were dissolved 12.1 g of diethyl 2(S)-hydroxy-3(R)-methyl-1,4-butanoate and 5.96 g of dihydropyran, and, after adding a catalytic amount of p-toluenesulfonic acid, the mixture was stirred for 6 hours at room temperature. The reaction liquor was washed with saturated aqueous solution of sodium hydrogencarbonate and then with saturated saline solution, dried over anhydrous Glauber's salt, and then concentrated. The residue was purified through silica gel column (elution solvent, n-hexane:ethyl acetate = 4:1) to obtain 16.5 g of diethyl 2(S)-[(2-tetrahydropyranyl)oxy]-3(R)-methyl-1,4-butanoate.

To a suspension of 150 ml of anhydrous ether and 3.24 g of aluminum lithium hydride, a solution of 50 ml of anhydrous ether and 16.5 g of diethyl 2(S)-[(2-tetrahydropyranyl)oxy]-3(R)-methyl-1,4-butanoate was added dropwise in a stream of argon under cooling with ice. After stirring for 2 hours at room temperature, 7 ml of water and then 5 ml of 15 % aqueous solution of sodium hydroxide were added under cooling with ice, and the insolubles were collected by filtration. The insolubles were washed thrice with 50 ml of THF. The filtrate and the washings were combined and concentrated to obtain 9.48 g of aimed product as colorless oil.

H - NMR (in $CDC1_3$, δ, ppm)

0.97, 0.99 (3H, dx2 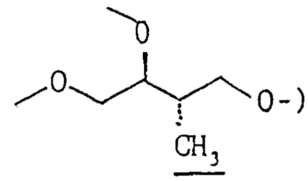

1.30-2.00 (6H, m 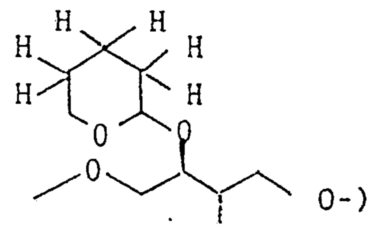

2.40-2.90 (1H, m 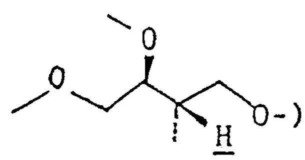

3.30-4.20 (9H, m 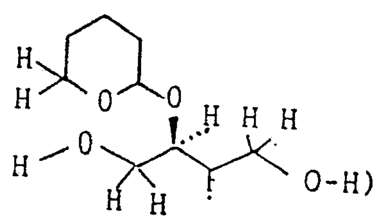

4.40-4.80 (1H, m 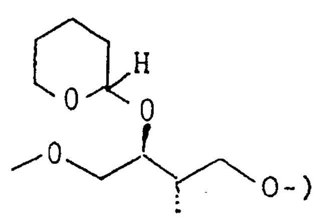

Referential Example 4

Synthesis of 2(R)-[(2-tetrahydropyranyl)oxy]-3(R)-methyl-1,4-butanediol

The reaction was performed similarly to Referential example 3 using D-malic acid as a raw material to obtain 15.2 g of aimed product.

H - NMR (in $CDC1_3$, δ, ppm)

0.97, 0.99 (3H, dx2

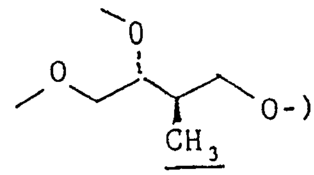

1.30-2.00 (6H, m

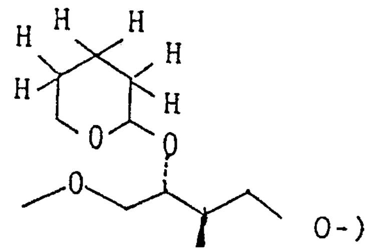

2.40-2.90 (1H, m

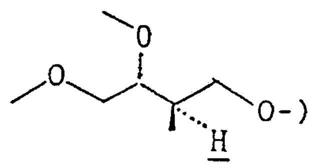

3.30-4.20 (9H, m

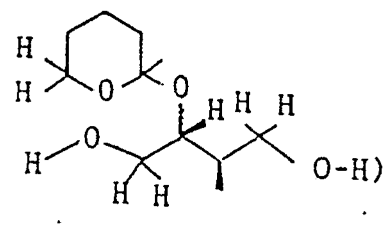

4.40-4.80 (1H, m

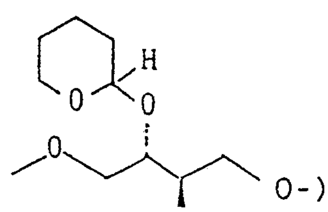

Example 1

Synthesis of 3(S)-[(t-butoxycarbonyl)amino]-2(S)-methyl-γ-butyrolactone

To a solution of 10 ml of anhydrous THF and 1.63 ml of diisopropylamine, 7.3 ml of n-butyllithium (1.6 M hexane solution) was added dropwise at 0 °C, and the mixture was stirred for 20 minutes. This solution was cooled to -78 °C and a solution of 10 ml of anhydrous THF and 704.3 mg of 3(S)-[(t-butoxycarbonyl)-amino]-γ-butyrolactone was added dropwise. This solution was added to a solution of 50 ml of anhydrous tetrahydrofuran and 6.5 ml of methyl iodide, which was cooled to -78 °C, and then the mixture was further stirred for 2 hours. To the reaction liquor was added 10 % aqueous solution of citric acid, and the solution was extracted with methylene chloride. The methylene chloride layer was washed in sequence with saturated aqueous solution of sodium hydrogencarbonate and saturated saline solution, dried over magnesium sulfate, and then concentrated. The residue was purified through silica gel column (elution solvent, n-hexane:ethyl acetate = 3:1 → 2.5:1) to obtain 580.5 mg of aimed product as colorless needle-like crystals and 83.2 mg of 3(S)-[(t-butoxycarbonyl)-amino]-2(R)-methyl-γ-butyrolactone.

Melting point: 139-140 °C

Specific rotation: $[\alpha]_D^{20}$ = -39.4 ° (c = 1.00, chloroform)

| Elemental analysis: $C_{10}H_{17}NO_4$ | | | |
|---|---|---|---|
| Calculated | C: 55.80 | H: 7.96 | N: 6.51 |
| Observed | C: 55.82 | H: 7.96 | N: 6.29 |

Example 2

Synthesis of 3(R)-[(t-butoxycarbonyl)amino]-2(R)-methyl-γ-butyrolactone

The reaction was performed through the similar process to Example 1 using 9.5 g of 3(R)-[(t-butoxycarbonyl)amino]-γ-butyrolactone to obtain 6.5 g of aimed product and 1.2 g of 3(R)-[(t-butoxycarbonyl)amino]-2(S)-methyl-γ-butyrolactone.

Melting point: 135-136 °C

Specific rotation: $[\alpha]_D^{20}$ = + 42.7° (c = 1.09, chloroform)

| Elemental analysis: $C_{10}H_{17}NO_4$ | | | |
|---|---|---|---|
| Calculated | C 55.80 | H : 7.96 | N : 6.51 |
| Observed | C 55.98 | H : 8.00 | N : 6.39 |

Example 3

Synthesis of 2(S)-[(t-butoxycarbonyl)amino]-3(S)-methylbutane-1,4-diol

Into 1 ml of THF were suspended 37.8 mg of sodium borohydride and 42.4 mg of lithium chloride, and, after a solution of 2 ml of ethanol and 107.6 mg of 3(S)-[(t-butoxycarbonyl)amino]-2(S)-methyl-γ-butyrolactone was added dropwise at -10 °C, the mixture was stirred for 7 hours at an internal temperature of 5 °C. To the reaction liquor was added 10% aqueous solution of citric acid, and the solution was then concentrated. The residue was diluted with saturated saline solution and extracted with ethyl acetate. The ethyl acetate layer was washed with saturated saline solution, dried over magnesium sulfate, and then concentrated to obtain 104.2 mg of aimed product as colorless crystals.

Melting point: 68-69.5 °C

Specific rotation: $[\alpha]_D^{20}$ = + 7.7° (C = 1.00, chloroform)

| Elemental analysis: $C_{10}H_{21}NO_4$ | | | |
|---|---|---|---|
| Calculated | C: 54.77 | H: 9.65 | N : 6.39 |
| Observed | C: 54.96 | H: 9.73 | N : 6.29 |

Example 4

Synthesis of 2(R)-[(t-butoxycarbonyl)amino]-3(R)-methyl-butane-1,4-diol

The reaction was performed similarly to Example 3 using 6.8 g of 3(R)-[(t-butoxycarbonyl)amino]-2(R)-methyl-γ-butyrolactone to obtain 4.0 g of aimed product.

Melting point: 64.5-66 °C

Specific rotation: $[\alpha]_D^{20}$ = -7.8° (c = 1.07, chloroform)

Elemental analysis: $C_{10}H_{21}NO_4$

| | | | |
|---|---|---|---|
| Calculated | C: 54.77 | H : 9.65 | N : 6.39 |
| Observed | C: 54.78 | H : 9.75 | N : 6.30 |

Example 5

Synthesis of 1-benzyl-3(S)-[(t-butoxycarbonyl)amino]-4(R)-methylpyrrolidine

Into 200 ml of anhydrous methylene chloride were dissolved 10.13 g of 2(S)-[(t-butoxycarbonyl)amino]-3(S)-methylbutane-1,4-diol and 14.2 ml of triethylamine, and, after 7.87 ml of methane sulfonyl chloride was added dropwise at -15 °C, the mixture was stirred for 45 minutes at the same temperature. The reaction liquor was washed with water and then with saturated saline solution, dried over magnesium sulfate, and then concentrated. To the residue were added 50 ml of benzylamine, and, after stirred for 2 hours at 60 °C, benzylamine was distilled off. The residue was purified through silica gel column to obtain 7.85 g of aimed product as colorless crystals.

Melting point: 58-60 °C

Specific rotation: $[\alpha]_D^{20}$ = -24.0° (c = 1.00, chloroform)

Example 6

Synthesis of 1-benzyl-3(R)-[(t-butoxycarbonyl)amino]-4(S)-methylpyrrolidine

The reaction was performed similarly to Example 5 using 2.3 g of 2(R)-[(t-butoxycarbonyl)amino]-3(R)-methylbutane-1,4-diol to obtain 1.2 g of aimed product.

Specific rotation: $[\alpha]_D^{20}$ = + 26.4° (c = 1.03, chloroform)

Example 7

Synthesis of 3(S)-[(t-butoxycarbonyl)amino]-4(R)-methyl-pyrrolidine

Into 40 ml of methanol were dissolved 3.07 g of 1-benzyl-3(S)-[(t-butoxycarbonyl)amino]-4(R)-methyl-pyrrolidine, and, after 0.16 g of 10 % palladium active charcoal and 8 ml of aqueous solution containing 2.67 g of ammonium formate were added, the mixture was refluxed for 2 hours at 80 °C. After filtering off the catalyst in the reaction liquor, this was concentrated and 10 g of sodium hydroxide were added to the residue. Ether was added to this and a procedure for collecting supernatant was repeated (100 ml in total). The ether layer was dried over sodium hydroxide and then concentrated. The residue was recrystallized from n-hexane to obtain 2.14 g of aimed product as colorless needle-like crystals.

Melting point: 95-96.5 °C

Specific rotation: $[\alpha]_D^{20}$ = 58.6° (c = 1.00, chloroform)

Elemental analysis: $C_{10}H_{20}N_2O_2$

| | | | |
|---|---|---|---|
| Calculated | C: 59.97 | H: 10.07 | N: 13.99 |
| observed | C: 59.90 | H: 10.05 | N: 13.91 |

Example 8

Synthesis of 3(R)-[(t-butoxycarbonyl)amino]-4(S)-methyl-pyrrolidine

The reaction was performed similarly to Example 7 using 1.20 g of 1-benzyl-3(R)-[(t-butoxycarbonyl)-

amino]-4(S)-methylpyrrolidine to obtain 0.74 g of aimed product.

Melting point: 95-96 °C

Specific rotation: $[\alpha]_D^{20}$ = + 58.5° (c = 1.09, chloroform)

Elemental analysis: $C_{10}H_{20}N_2O_2$

| | | | |
|---|---|---|---|
| Calculated | C: 59.97 | H: 10.07 | N: 13.99 |
| Observed | C: 60.03 | H: 10.07 | N: 13.97 |

Example 9

Synthesis of 2(S)-[(2-tetrahydronyranyl)oxy]-3(S)-methyl-1,4-di(p-toluenesulfonyloxy) butane

To a solution of 15 ml of pyridine and 2.56 g of 2(S)-[(2-tetrahydropyranyl)oxy]-3(S)-methyl-1,4-butanediol were added gradually 5.24 g of p-toluenesulfonyl chloride under cooling with ice, and then the mixture was stirred for 1 hour at the same temperature and further for 1 hour at room temperature. The reaction liquor was poured into ice water, which was extracted with ethyl acetate. The ethyl acetate layer was washed with water and then with saturated saline solution, dried over anhydrous Glauber's salt, and then concentrated. The residue was purified through silica gel column (elution solvent, chloroform) to obtain 4.63 g of aimed product as colorless oil.

H - NMR (in $CDCl_3$, δ, ppm)

0.87, 0.89 (3H, dx2 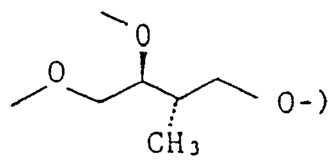

1.20-1.80 (6H, m 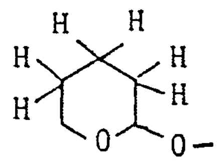

1.90-2.20 (1H, m 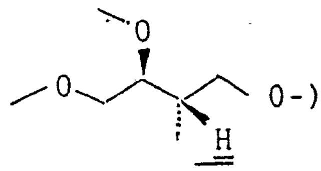

2.44 (6H, S, $O_2$ S- 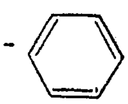 -$CH_3$ x 2)

3.20-4.20 (7H, m 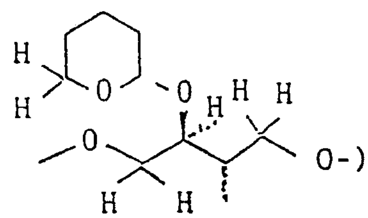

4.30-4.60 (1H, m 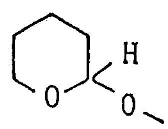

7.32-7.76 (8H, m 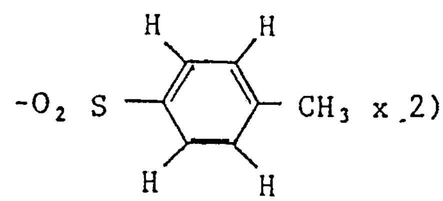

Example 10

Synthesis of 2(R]-[(2-tetrahydronyranyl]oxy]-3(R)-methyl-1,4-di(p-toluenesulfonyloxy)butane

The reaction was performed similarly to Example 9 using 15.0 g of 2(R)-[(2-tetrahydropyranyl)oxy]-3(R)-methyl-1,4-butanediol to obtain 27.4 g of aimed product.

H - NMR (in $CDCl_3$, δ, ppm)

0.87, 0.89 (3H, dx2 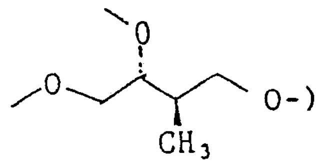

1.20-1.80 (6H, m 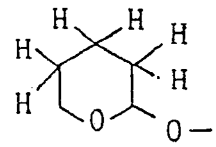

1.90-2.20 (1H, m 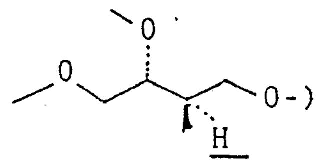

2.44 (6H, S, $O_2$ S - 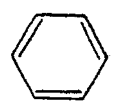 - $CH_3$ x2)

3.20-4.20 (7H, m 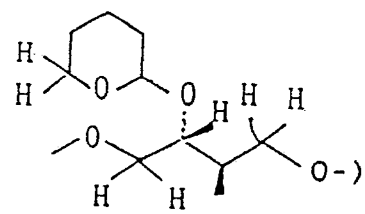

4.30-4.60 (1H, m 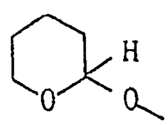

7.32-7.76 (8H, m - $O_2$ S 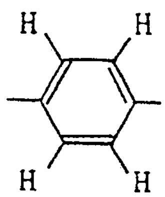 $CH_3$ x2)

Example 11

Synthesis of 1,4-dibromo-2(S)-hydroxy-3(R)-methylbutane

To 200 ml of acetone were added 12.2 g of 2(S)-[(2-tetrahydropyranyl)oxy]-3(S)-methyl-1,4-di(p-toluenesulfonyloxy)butane and 25.0 g of lithium bromide, and the mixture was stirred for 6 hours under heat. The reaction liquor was concentrated and the residue was dissolved into 100 ml of ethyl acetate. The insolubles were filtered off and the ethyl acetate layer was washed with water, dried over anhydrous Glauber's salt, and then concentrated to obtain 6.50 g of aimed product as light red oil.

H - NMR (in $CDCl_3$, δ, ppm)

1.05 (3H, d 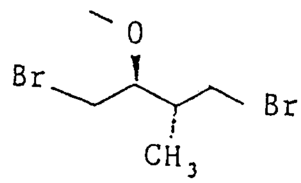 )

1.80-2.20 (1H, m 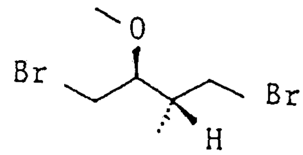

2.20-2.50 (1H, br 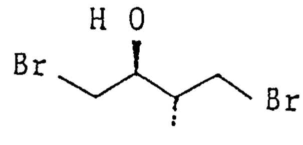 )

3.40-3.90 (5H, m 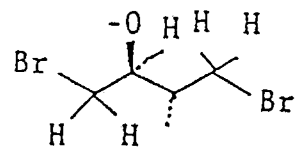 )

Example 12

Synthesis of 1,4-dibromo-2(R)-hydroxy-3(S)-methylbutane

The reaction was performed similarly to Example 11 using 22.0 g of 2(R)-[(2-tetrahydropyranyl)oxy]-3-(R)-methyl-1,4-di-(p-toluenesulfonyloxy)butane to obtain 10.5 g of aimed product.

H - NMR (in $CDCl_3$, δ, ppm)

1.05 (3H, d 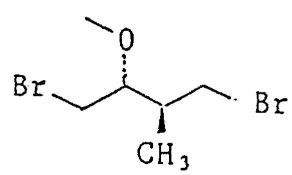 )

1.80-2.20 (1H, m 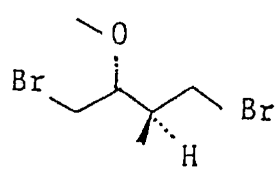

2.20-2.50 (1H, br 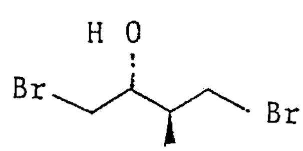 )

3.40-3.90 (5H, m 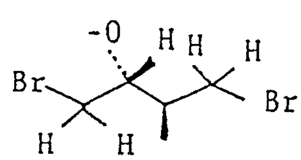 )

Example 13

Synthesis of 1,4-dibromo-2(S)-[(2-tetrahydropyranyl)oxy]-3(R)-methylbutane

Into 30 ml of anhydrous ether were dissolved 6.50 g of 1,4-dibromo-2(S)-hydroxy-3(R)-methylbutane and 2.40 g of dihydropyran, and, after a catalytic amount of p-toluenesulfonic acid was added, the mixture was stirred overnight at room temperature. The reaction liquor was washed with saturated aqueous solution of sodium hydrogencarbonate and then with saturated saline solution, dried over anhydrous Glauber's salt, and then concentrated to obtain 7.86 g of aimed product as pale yellow oil.

H - NMR (in $CDCl_3$, $\delta$, ppm)

1.01, 1.07 (3H, dx2 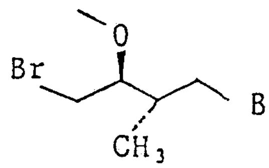 )

1.30-2.00 (6H, m 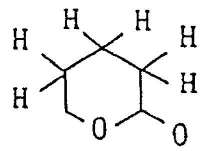

2.00-2.60 (1H, m 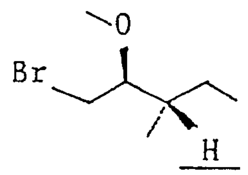 )

3.40-4.20 (7H, m 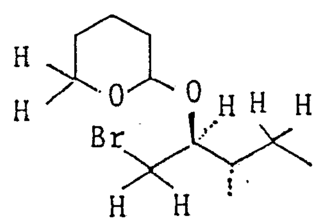 )

4.70-5.10 (1H, m

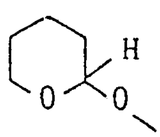 )

Example 14

Synthesis of 1,4-dibromo-2(R)-[(2-tetrahydropyranyl)oxy]-3(S)-methylbutane

The reaction was performed similarly to Example 13 using 16.9 g of 1,4-dibromo-2(R)-hydroxy-3(S)-methylbutane to obtain 21.2 g of aimed product.

H - NMR (in $CDCl_3$, $\delta$, ppm)

1.07, 1.07 (3H, dx2 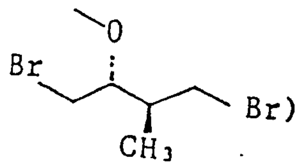

1.30-2.00 (6H, m 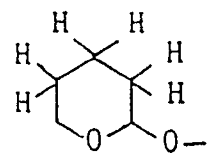

2.00-2.60 (1H, m 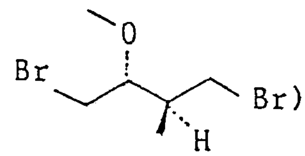

3.40-4.20 (7H, m 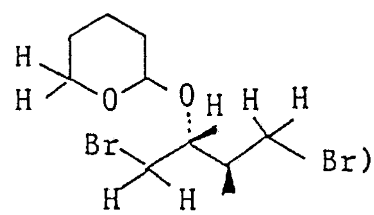

4.70-4.90 (1H, m

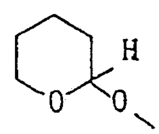

Example 15

Synthesis of 1-benzyl-3(S)-[(2-tetrahydropyranyl)oxyl-4(S)-methylpyrrolidine

a) To a solution of 5.5 ml of water containing 1.09 g of sodium hydroxide was added a solution of 8.3 ml of toluene containing 1.42 g of benzylamine and 4.00 g of 2(S)-[(2-tetrahydropyranyl)-oxy]-3(S)-methyl-1,4-di(p-toluenesulfonyloxy)butane, and the mixture was stirred for 3 hours at 65° C and further for 2 hours at 80° C. To the reaction liquor was added 30 ml of water and 50 ml of benzene, and the organic layer was separated. The water layer was extracted further with 30 ml of benzene. The organic layers were combined, washed with water and then saturated saline solution, dried over anhydrous Glauber's salt, and then concentrated. The residue was purified through silica gel column (elution solvent, chloroform → chloroform:methanol = 10:1) to obtain 1.00 g of aimed product as yellow oil.

b) To 15 ml of isopropyl alcohol were added 1.00 g of 2(S)-[(2-tetrahydropyranyl)oxy]-3(S)-methyl-1,4-di-(p-toluenesulfonyloxy)butane, 0.32 g of benzylamine and 0.81 g of potassium carbonate, and the mixture was refluxed for 5 hours under heat. The insolubles in reaction liquor were filtered off and the filtrate was concentrated. The residue was purified through silica gel column (elution solvent, chloroform:methanol = 10:1) to obtain 0.22 g of aimed product.

c) To 140 ml of isopropyl alcohol were added 7.86 g of 1,4-dibromo-2(S)-[(2-tetrahydropyranyl)oxy]-3(R)-methylbutane, 5.10 g of benzylamine and 13.2 g of potassium carbonate, and the mixture was refluxed for 8 hours under heat. The insolubles in reaction liquor were filtered off and the filtrate was concentrated. The residue was purified through silica gel column (elution solvent, chloroform:methanol = 10:1) to obtain 5.33 g of aimed product.

H - NMR (in $CDCl_3$)

0.97, 1.08 (3H, dx2 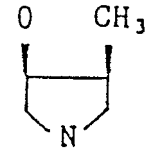

1.40-1.80 (6H, m 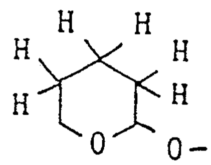

2.01-3.47 (5H, m 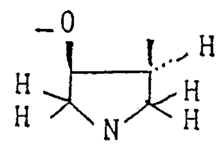

3.61-3.64 (2H, Sx2 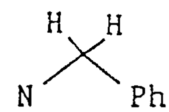

3.60-4.00 (2H, m 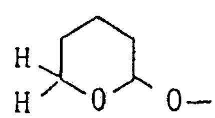

4.10-4.40 (1H, m 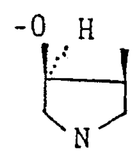

4.50-4.70 (1H, m 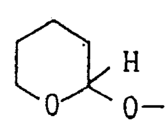

7.28 (5H, S 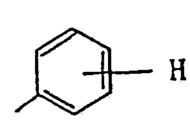

Example 16

Synthesis of 1-benzyl-3[R)-[(2-tetrahydropyranyl)oxy]-4(R)-methylpyrrolidine

The reaction was performed similarly to Example 15-c using 31.1 g of 1,4-dibromo-2(R)-[(2-tetrahydropyranyl)oxy]-3(S)-methylbutane to obtain 13.8 g of aimed product.

H - NMR (in $CDCl_3$, δ, ppm)

0.97-1.08 (3H, dx2

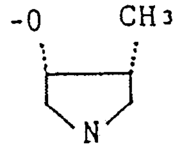

1.40-1.80 (6H, m

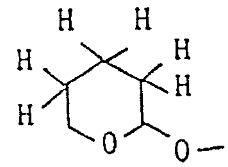

2.01-3.32 (5H, m

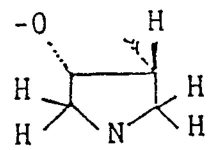

3.60-4.00 (2H, m

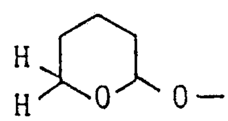

3.61-3.64 (2H, Sx2

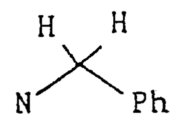

4.10-4.40 (1H, m

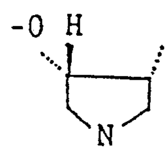

4.50-4.70 (1H, m

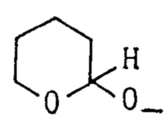

7.28 (5H, S

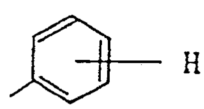

Example 17

Synthesis of 1-benzyl-3(S)-hydroxy-4(S)-methylpyrrolidine

a) Into 15 ml of methanol were dissolved 1.41 g of 1-benzyl-3(S)-[(2-tetrahydropyranyl)oxy]-4(S)-methylpyrrolidine, and, after 0.7 ml of concentrated hydrochloric acid was added, the mixture was stirred for 2 hours at room temperature. The reaction liquor was concentrated and 20 ml of water were added to

the residue, which was neutralized with aqueous solution of sodium hydroxide and extracted with ether. The ether layer was washed with saturated saline solution, dried over anhydrous Glauber's salt, and then concentrated to obtain 0.80 g of aimed product as red oil.

b) To 500 ml of isopropyl alcohol were added 28.9 g of 1,4-dibromo-2(S)-hydroxy-3(R)-methylbutane, 25.3 g of benzylamine and 65.2 g of potassium carbonate, and the mixture was refluxed for 2 hours under heat. The insolubles in the reaction liquor were filtered off and the filtrate was concentrated. The residue was dissolved into 300 ml of benzene, washed with water and then with saturated saline solution, dried over anhydrous Glauber's salt, and then concentrated. The residue was distilled under reduced pressure and the fraction at 110 to 160° C (0.7-1.0 mmHg) was further purified through silica gel column (elution solvent, chloroform:methanol = 10:1) to obtain 6.08 g of aimed product.

Specific rotation: $[\alpha]_D^{20}$ = -9.19° (c = 1.54, chloroform)

H - NMR (in $CDCl_3$, δ, ppm)

1.02 (3H, d 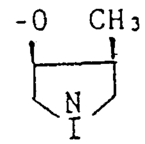

2.10-2.44 (3H, m 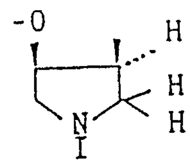

2.49-3.00 (2H, m 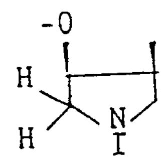

3.64 (2H, S 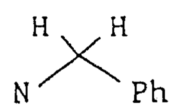

4.00-4.24 (1H, m 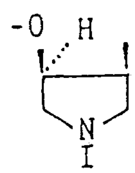

7.29 (5H, S 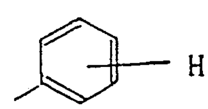

Example 18

Synthesis of 1-benzyl-3(R)-hydroxy-4(R)-methylpyrrolidine

a) The reaction was performed similarly to Example 17-a using 22.6 g of 1-benzyl-3(R)-[(2-tetrahydropyranyl)oxy]-4(R)-methylpyrrolidine to obtain 13.6 g of aimed product.

b) The reaction was performed similarly to Example 17-b using 1.5 g of 1,4-dibromo-2(R)-hydroxy-3(S)-methylbutane to obtain 0.58 g of aimed product.

Specific rotation: $[\alpha]_D^{20}$ = +10.8° (c = 2.68, chloroform)

H - NMR (in $CDCl_3$, δ, ppm)

1.01 (3H, d 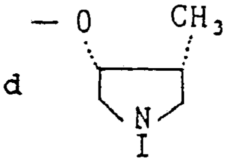 )

1.90-2.20 (1H, br 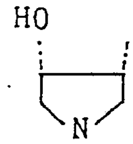 )

2.16-2.99 (5H, m 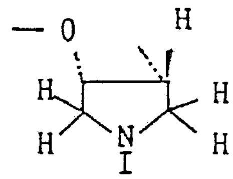

3.64 (2H, S 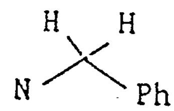

4.00-4.24 (1H, m 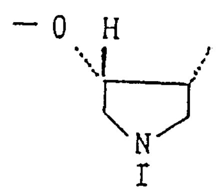

7.28 (5H, S 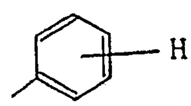

Example 19

Synthesis of 1-benzyl-3(R)-hydroxy-4(S)-methylpyrrolidine

Into 50 ml of anhydrous THF were dissolved 5.31 g of 1-benzyl-3(S)-hydroxy-4(S)-methylpyrrolidine, 8.75 g of triphenylphosphine and 4.07 g of benzoic acid, and 5.81 g of diethyl azodicarboxylate were added dropwise below 0° C in a stream of argon while cooling in a salt-ice bath. After stirring overnight at room temperature, the reaction liquor was concentrated. Ether was added to the residue, triphenylphosphine oxide deposited was filtered off, and the filtrate was concentrated. The residue was purified through silica gel column (elution solvent, n-hexane:ethyl acetate = 4:1) to obtain 4.62 g of 3(R)-benzoyloxy-1-benzyl-4(S)-methylpyrrolidine as yellow oil.

Into 25 ml of ethanol were dissolved 2.59 g of 3(R)-benzoyloxy-1-benzyl-4(S)-methylpyrrolidine, and, after 1.82 g of potassium carbonate was added, the mixture was refluxed for 4 hours under heat. The reaction liquor was concentrated and water was added to the residue, which was extracted with ether and then with benzene. The organic layers were combined, dried over anhydrous Glauber's salt and then concentrated. The residue was purified through silica gel column (elution solvent, chloroform:methanol = 10:1) to obtain 1.42 g of aimed product as light orange oil.

Specific rotation: $[\alpha]_D^{20}$ = +14.3° (c = 2.08, chloroform)

H - NMR (in $CDCl_3$, δ, ppm)

1.01 (3H, d 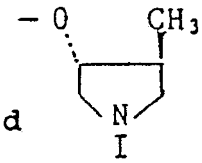 )

2.20-2.80 (1H, m 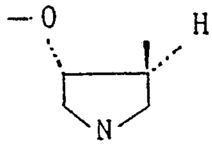 )

1.76-1.93, 2.39-3.08 (4H, m 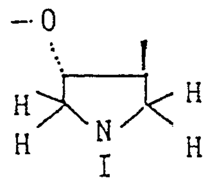

3.53 (2H, S 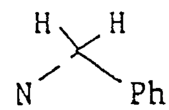

3.70-3.84 (1H, m 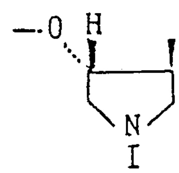

3.90-4.10 (1H, br 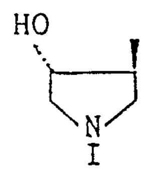 )

7.25 (5H, S 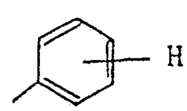

Example 20

Synthesis of 1-benzyl-3(S)-hydroxy-4(R)-methylpyrrolidine

The reaction was performed similarly to Example 19 using 10.58 g of 1-benzyl-3(R)-hydroxy-4(R)-methylpyrrolidine to obtain 6.11 g of aimed product.

Specific rotation: $\{\alpha]_D^{20}$ = -13.7° (c=2.03, chloroform)

Example 21

Synthesis of 3(S)-azido-l-benzyl-4(S)-methylpyrrolidine

a) Into 15 ml of pyridine were dissolved 2.86 g of 1-benzyl-3(R)-hydroxy-4(S)-methylpyrrolidine, and 3.14

g of p-toluenesulfonyl chloride were added gradually below 0 °C while cooling in a salt-ice bath. After stirring for 3 hours below 0 °C, ice water and saturated aqueous solution of sodium hydrogen carbonate were added to the reaction liquor, which was extracted with benzene. The benzene layer was washed with saturated saline solution, dried over anhydrous Glauber's salt, and then concentrated. The residue was purified through silica gel column (elution solvent, chloroform) to obtain 3.76 g of 1-benzyl-4(S)-methyl-3(R)-toluenesulfonyloxy-pyrrolidine as yellow oil.

Into 35 ml of dimethylformamide were dissolved 3.76 g of 1-benzyl-4(S)-methyl-3(R)-toluenesulfonyloxypyrrolidine, and after 3.54 g of sodium azide was added, the mixture was stirred for 3 hours at 90 °C. The reaction liquor was concentrated and water was added to the residue, which was extracted with benzene and then with ethyl acetate. The organic layers were combined, washed with saturated saline solution, dried over anhydrous Glauber's salt, and then concentrated. The residue was purified through silica gel column (elution solvent, chloroform) to obtain 2.09 g of aimed product as yellow oil.

b) Into 70 ml of anhydrous methylene chloride were dissolved 7.17 g of 1-benzyl-3(R)-hydroxy-4(S)-methylpyrrolidine, and, after 15.4 g of triethylamine and then 8.69 g of methanesulfonyl chloride were added while cooling in a dry ice-acetone bath, the mixture was stirred for 15 minutes at the same temperature. Water was added to the reaction liquor, the methylene chloride layer was separated, and the water layer was further extracted with methylene chloride. The methylene chloride layers were combined, washed with water, saturated aqueous solution of sodium hydrogen carbonate and saturated saline solution in this order, dried over anhydrous Glauber's salt, and then concentrated to obtain 9.85 g of 1-benzyl-3(R)-methanesulfonyloxy-4(S)-methylpyrrolidine.

Into 80 ml of acetonitrile were dissolved 9.85 g of 1-benzyl-3(R)-methanesulfonyloxy-4(S)-methylpyrrolidine, and, after 26.0 g of tetra-n-butyl ammonium azide were added, the mixture was stirred for 1 hour at 50 to 60 °C. The reaction liquor was concentrated and water was added to the residue, which was extracted with ethyl acetate. The ethyl acetate layer was washed with water, dried over anhydrous Glauber's salt, and then concentrated. The residue was purified through silica gel column (elution solvent, n-hexane:ethyl acetate = 4:1) to obtain 6.38 g of aimed product as yellow oil.

Specific rotation: $[\alpha]_D^{20}$ = -29.9° (c = 1.83, chloroform)

IR (liquid film): 2100 $cm^{-1}$ ($-N_3$)

H = NMR (in $CDCl_3$, $\delta$, ppm)

1.08 (3H, d 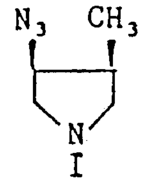 )

2.12-3.19 (5H, m 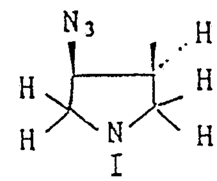 )

3.63 (2H, s 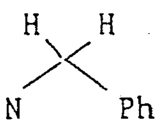 )

3.81-4.10 (1H, m 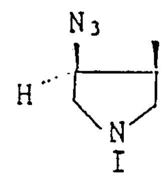

7.28 (5H, s 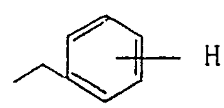

Example 22

Synthesis of 3(R)-azido-1-benzyl-4(R)-methylpyrrolidine

The reaction was performed similarly to Example 21-a using 6.11 g of 1-benzyl-3(S)-hydroxy-4(R)-methylpyrrolidine to obtain 5.98 g of aimed product as yellow oil.

Specific rotation: $[\alpha]_D^{20}$ = +24.8° (c = 3.60, chloroform)

IR (liquid film): 2100 $cm^{-1}$ ($-N_3$)

Example 23

Synthesis of 3(R)-azido-1-benzyl-4(S)-methylpyrrolidine

The reaction was performed similarly to Example 21-b using 5.00 g of 1-benzyl-3(S)-hydroxy-4(S)-methylpyrrolidine to obtain 4.52 g of aimed product as yellow oil.

Specific rotation: $[\alpha]_D^{20}$ = + 47.1 (c = 1.60, chloroform)

Example 24

Synthesis of 3(S)-azido-1-benzyl-4(R)-methylpyrrolidine

The reaction was performed similarly to Example 21-b using 5.00 g of 1-benzyl-3(R)-hydroxy-4(R)-methylpyrrolidine to obtain 5.27 g of aimed product as yellow oil.

Specific rotation: $[\alpha]_D^{20}$ = -50.5 (c = 1.11, chloroform)

Example 25

Synthesis of 1-benzyl-3(S)-[(t-butoxycarbonyl)amino]-4(S)-methylpyrrolidine

Into 90 ml of toluene were dissolved 20 ml (70 % toluene solution) of sodium bis (2-methoxyethoxy) aluminum hydride, and, after a solution of 15 ml of toluene containing 6.38 g of 3(S)-azido-l-benzyl-4(S)-methylpyrrolidine was added dropwise at an internal temperature of below 10 °C, the mixture was stirred for 30 minutes at room temperature. Ice pieces and an aqueous solution of sodium hydroxide were added to the reaction liquor, the toluene layer was separated, and the aqueous layer was extracted with benzene. The toluene layer and the benzene layer were combined, dried over anhydrous Glauber's salt, and then concentrated. The residue was distilled under reduced pressure (140 °C, 0.4 mmHg) to obtain 4.71 g of 3-(S)-amino-l-benzyl-4(S)-methylpyrrolidine as colorless oil.

Into 15 ml of acetonitrile were dissolved 4.71 g of 3(S)-amino-1-benzyl-4(S)-methylpyrrolidine, and, after 5.68 g of di-t-butyl dicarbonate were added, the mixture was stirred for 1 hour at room temperature and then it was allowed to stand for 3 hours in a refrigerator (0 °C). The crystals deposited were collected by filtration and washed with n-hexane to obtain 5.27 g of aimed product as white crystals.

Melting point: 98-100 °C

Specific rotation: $[\alpha]_D^{20}$ = +31.0° (c = 1.00, chloroform)

Elemental analysis: $C_{10}H_{26}N_2O_2$

| | | | |
|---|---|---|---|
| Calculated | C : 70.31 | H : 9.02 | N : 9.65 |
| Observed | C : 70.36 | H : 8.96 | N : 9.86 |

Example 26

Synthesis of 1-benzyl-3(R)-[(t-butoxycarbonyl)amino]-4(R)-methylpyrrolidine

The reaction was performed similarly to Example 25 using 5.98 g of 3(R)-azido-l-benzyl-4(R)-methylpyrrolidine to obtain 3.08 g of aimed product.

Melting point: 95-96 °C

Specific rotation: $[\alpha]_D^{20}$ = -29.9° (c = 1.95, chloroform)

Example 27

Synthesis of 1-benzyl-3(R)-[(t-butoxycarbonyl)amino]-4(S)-methylpyrrolidine

The reaction was performed similarly to Example 25 using 5.00 g of 3(R)-azido-1-benzyl-4(S)-methylpyrrolidine to obtain 3.53 g of aimed product.

Melting point: 76-77 °C

Specific rotation: $[\alpha]_D^{20}$ = +27.4° (c = 1.39, chloroform)

Elemental analysis: $C_{17}H_{26}N_2O_2$

| | | | |
|---|---|---|---|
| Calculated | C : 70.31 | H : 9.02 | N : 9.65 |
| Observed | C : 70.55 | H : 9.12 | N : 9.69 |

Example 28

Synthesis of 1-benzyl-3(S)-[(t-butoxycarbonyl)amino]-4(R)-methylpyrrolidine

The reaction was performed similarly to Example 25 using 5.21 g of 3(S)-azido-1-benzyl-4(R)-methyl-

pyrrolidine to obtain 4.31 g of aimed product.

Melting point: 75-76° C

Specific rotation: $[\alpha]_D^{20}$ = - 27.9° (c = 1.09, chloroform)

Elemental analysis: $C_{17}H_{26}N_2O_2$

| | | | |
|---|---|---|---|
| Calculated | C : 70.31 | H : 9.02 | N : 9.65 |
| Observed | C : 70.45 | H : 9.08 | N : 9.70 |

Example 29

Synthesis of 3(S)-[(t-butoxycarbonyl)amino]-4(S)-methylpyrrolidine

Into 60 ml of methanol were dissolved 5.27 g of 1-benzyl-3(S)-[(t-butoxycarbonyl)amino]-4(S)-methylpyrrolidine, and, after 12 ml of water containing 0.28 g of suspended 10% palladium charcoal and 4.57 g of dissolved ammonium formate was added, the mixture was refluxed for 1 hour at 80° C. The catalyst in reaction liquor was filtered off and the filtrate was concentrated. After 12.0 g of potassium hydroxide was added to the residue, water and ether were added to extract. The ether layer was separated, washed with saturated saline solution, dried over anhydrous Glauber's salt, and then concentrated. The residue thus obtained was recrystallized from n-hexane to obtain 3.19 g of aimed product as white needle-like crystals.

Melting point: 84-85° C

Specific rotation: $[\alpha]_D^{20}$ = +18.9° (c = 1.47, chloroform)

Elemental analysis: $C_{10}H_{20}N_2O_2$

| | | | |
|---|---|---|---|
| Calculated | C : 59.97 | H : 10.07 | N : 13.99 |
| Observed | C : 59.62 | H : 10.07 | N : 13.78 |

Example 30

Synthesis of 3(R)-[(t-butoxycarbonyl)amino]-4(R)-methylpyrrolidine

The reaction was performed similarly to Example 29 using 1.35 g of 1-benzyl-3(R)-[(t-butoxycarbonyl)-amino]-4(R)-methylpyrrolidine to obtain 0.63 g of aimed product.

Melting point: 83-84° C

Specific rotation: $[\alpha]_D^{20}$ = -18.7° (c = 1.67, chloroform)

Elemental analysis: $C_{10}H_{20}N_2O_2$

| | | | |
|---|---|---|---|
| Calculated | C : 59.97 | H : 10.07 | N : 13.99 |
| Observed | C : 59.46 | H : 9.97 | N : 13.90 |

Example 31

Synthesis of 3(R)-[(t-butoxycarbonyl)amino]-4(S)-methylpyrrolidine

The reaction was performed similarly to Example 29 using 1.50 g of 1-benzyl-3(R)-[(t-butoxycarbonyl)-amino]-4(S)-methylpyrrolidine to obtain 0.75 g of aimed product.

Melting point: 92-94 °C
Specific rotation: $[\alpha]_D^{20}$ = +56.9° (c=1.05, chloroform)

Elemental analysis: $C_{10}H_{20}N_2O_2$

| | | | |
|---|---|---|---|
| Calculated | C : 59.97 | H : 10.07 | N : 13.99 |
| Observed | C : 59.81 | H : 10.06 | N : 13.75 |

Example 32

Synthesis of 3(S)-[(t-butoxycarbonyl)amino]-4(R)-methylpyrrolidine

The reaction was performed similarly to Example 29 using 2.00 g of 1-benzyl-3(S)-[(t-butoxycarbonyl)-amino]-4(R)-methylpyrrolidine to obtain 1.26 g of aimed product.
Melting point: 92-93 °C
Specific rotation: $[\alpha]_D^{20}$ = -56.8° (c=1.29, chloroform)

Elemental analysis: $C_{10}H_{20}N_2O_2$

| | | | |
|---|---|---|---|
| Calculated | C : 59.97 | H : 10.07 | N : 13.99 |
| Observed | C : 60.32 | H : 10.19 | N : 13.78 |

Example 33

Synthesis of 7-[3(S)-amino-4(R)-methyl-l-pyrrolidinyl]-1-cyclopropyl-6-fluoro-1,4-dihydro-8-methoxy-4-oxo-3-quinolinecarboxylic acid hydrochloride

To 30 ml of anhydrous acetonitrile were added 3.69 g of 1-cyclopropyl-6,7-difluoro-1,4-dihydro-8-methoxy-4-oxo-3-quinolinecarboxylic acid, 3.02 g of 3(S)-[(t-butoxycarbonyl) amino]-4(R)-methylpyrrolidine and 1.90 g of 1,8-diazabicyclo-[5,4,0]-7-undecene (DBU), and the mixture was refluxed for 8 hours at 100 °C. The reaction liquor was concentrated and the residue was dissolved into 150 ml of methylene chloride, which was washed with 10 % aqueous solution of citric acid and then with saturated saline solution, dried over magnesium sulfate, and then concentrated. The residue was purified through silica gel column (elution solvent, chloroform:methanol: concentrated aqueous ammonia=50:10:1) to obtain 5.05 g of 7-[3(S)-[(t-butoxycarbonyl)amino]-4(R)-methyl-l-pyrrolidinyl]-l-cyclopropyl-6-fluoro-1,4-dihydro-8-methoxy-4-oxo-3-quinolinecarboxylic acid. Into 5 ml of ethanol were dissolved 3.60 g of 7-[3(S)-[t-butoxycarbonyl)-amino]-4(R)-methyl-1-pyrrolidinyl]-1-cyclopropyl-6-fluoro-1,4-dihydro-8-methoxy-4-oxo-quinolonecarboxylicacid, and, after 5 ml of hydrochloric acid/ethanol was added, the mixture was stirred for 2 hours at room temperature. The crystals deposited were collected by filtration, washed with ether and then recrystallized from ethanol to obtain 2.45 g of aimed product as pale yellow crystals.
Melting point: 200-202 °C
Specific rotation: $[\alpha]_D^{20}$ = +124.1° (c=1.00, DMSO)

Elemental analysis: $C_{19}H_{22}FN_3O_4$ . H Cl . 1/2 $H_2O$

| | | | |
|---|---|---|---|
| Calculated | C : 54.22 | H : 5.75 | N : 9.98 |
| Observed | C : 54.40 | H : 5.87 | N : 9.70 |

Example 34

Synthesis of 7-[3(S)amino-4(S)-methyl-1-pyrrolidinyl]-1-cyclopropyl-6-fluoro-1,4-dihydro-8-methoxy-4-oxo-3-quinolinecarboxyic acid hydrochloride

To 20 ml of acetonitrile were added 2.00 g of 1-cyclopropyl-6,7-difluoro-1,4-dihydro-8-methoxy-4-oxo-3-quinolinecarboxylic acid, 2.15 g of 3(S)-[(t-butoxycarbonyl)amino]-4(S)-methyl-pyrrolidine and 1.09 g of DBU, and the mixture was refluxed for 7 hours at 100°C. The reaction liquor was concentrated and the residue was dissolved into 100 ml of chloroform, which was washed with 10% aqueous solution of citric acid, water and saturated saline solution in this order, dried over anhydrous Glauber's salt, and then concentrated. The residue was purified through silica gel column (elution solvent, chloroform:methanol: concentrated aqueous ammonia = 20:5:1) to obtain 3.06 g of 7-[3(S)-[(t-butoxycarbonyl)amino]-4(S)-methyl-l-pyrrolidinyl]-l-cyclopropyl-6-fluoro-1,4-dihydro-8-methoxy-4-oxo-3-quinolinecarboxylic acid.

Into 5 ml of ethanol were dissolved 3.06 g of 7-[3(S)-[(t-butoxycarbonyl)amino]-4(S)-methyl-1-pyrrolidinyl]-l-cyclopropyl-6-fluoro-1,4-dihydro-8-methoxy-4-oxo-3-quinolinecarboxylic acid, and, after 2 ml of hydrochloric acid/ethanol was added, the mixture was stirred for 15 minutes at 50°C. After cooling, ether was added to the reaction liquor and the crystals deposited were collected by filtration. The crystals collected by filtration were recrystallized from a mixed liquor of isopropyl alcohol-ethanol-ether to obtain 2.37 g of aimed product as yellow powder.

Melting point: 185-192°C (decomposed gradually)

Specific rotation: $[\alpha]_D^{20}$ = -92.3° (c = 1.04, DMSO)

| Elemental analysis: | $C_{19}H_{22}FN_3O_4$ . H Cl . 3/4 $H_2O$ | | |
|---|---|---|---|
| Calculated | C : 53.65 | H : 5.81 | N : 9.88 |
| Observed | C : 53.81 | H : 6.20 | N : 9.30 |

Example 35

Synthesis of 7-[3(R)-amino-4(R)-methyl-1-pyrrolidinyl]-1-cyclopropyl-6-fluoro-1,4-dihydro-8-methoxy-4-oxo-3-quinolinecarboxylic acid hydrochloride

The reaction was performed similarly to Example 34 using 1.03 g of 1-cyclopropyl-6,7-difluoro-1,4-dihydro-8-methoxy-4-oxo-3-quinolinecarboxylic acid and 0.89 g of 3(R)-[(t-butoxycarbonyl)amino]-4(R)-methylpyrrolidine to obtain 0.63 g of aimed product.

Melting point: 175-184°C (decomposed gradually)

Specific rotation: $[\alpha]_D^{20}$ = +85.1° (c = 1.07, DMSO)

| Elemental analysis: | $C_{19}H_{22}FN_3O_4$ . H Cl . 3/2$H_2O$ | | |
|---|---|---|---|
| Calculated | C : 51.99 | H : 5.97 | N : 9.57 |
| Observed | C : 52.15 | H : 5.97 | N : 8.63 |

Example 36

Synthesis of 7-[3(R)-amino-4(S)-methyl-l-pyrrolidinyl]-l-cyclopropyl-6-fluoro-1-4-dihydro-8-methoxy-4-oxo-3-quinolinecarboxylic acid hydrochloride

The reaction was performed similarly to Example 33 using 639 mg of 1-cyclopropyl-6,7-difluoro-1,4-dihydro-8-methoxy-4-oxo-3-quinolinecarboxylic acid and 650 mg of 3(R)-[(t-butoxycarbonyl)amino]-4(S)-methylpyrrolidine to obtain 350 mg of aimed product.

Melting point: 195-197 °C
Specific rotation: $[\alpha]_D^{20}$ = - 126.9° (c=1.10, DMSO)

Elemental analysis: $C_{19}H_{22}FN_3O_4$ . H Cl . 3/4$H_2$ O

| | | | |
|---|---|---|---|
| Calculated | C : 53.65 | H : 5.68 | N : 9.88 |
| Observed | C : 53.77 | H : 5.55 | N : 9.88 |

Test Example 1 - Antibacterial spectra

The antibacterial test was performed according to the method designated by Society of Chemotherapy in Japan. The results are shown in Table 1.

Table 1 Antibacterial Spectra

| Organism tested ($10^6$ cells/ml) | Gram | Minimum inhibitory concentration for growth (μg/ml) | | | | |
|---|---|---|---|---|---|---|
| | | Example 33 | Example 34 | Example 35 | Example 36 | CPFX |
| S. aureus 209P | + | 0.025 | 0.025 | 0.025 | 0.05 | 0.20 |
| S. aureus Smith | + | 0.025 | 0.025 | 0.025 | 0.10 | 0.39 |
| S. epidermidis IID866 | + | 0.05 | 0.05 | 0.05 | 0.10 | 0.20 |
| S. pyogenes S-8 | + | 0.05 | 0.10 | 0.20 | 0.20 | 0.78 |
| S. pyogenes IID692 | + | 0.10 | 0.10 | 0.39 | 0.39 | 0.78 |
| S. pneumoniae type III | + | 0.05 | 0.05 | 0.10 | 0.20 | 0.39 |
| S. pneumoniae S-4288 | + | 0.10 | 0.20 | 0.20 | 0.39 | 0.78 |
| S. pneumoniae IID552 | + | 0.10 | 0.20 | 0.20 | 0.39 | 0.78 |
| E. faecalis IID682 | + | 0.10 | 0.20 | 0.20 | 0.39 | 0.78 |
| E. coli NIHJ JC-2 | – | 0.0125 | 0.0125 | 0.025 | 0.025 | 0.0063 |
| K. pneumoniae KY6445 | – | 0.025 | 0.025 | 0.05 | 0.05 | 0.0125 |
| E. cloacae IID977 | – | 0.05 | 0.10 | 0.10 | 0.20 | 0.025 |
| C. freundii IID976 | – | 0.05 | 0.05 | 0.10 | 0.10 | 0.0125 |
| P. vulgaris IFO3167 | – | 0.0125 | 0.025 | 0.025 | 0.025 | 0.0125 |
| P. mirabilis IID994 | – | 0.05 | 0.025 | 0.05 | 0.10 | 0.025 |
| M. morganii IID602 | – | 0.10 | 0.10 | 0.10 | 0.20 | 0.025 |
| S. marcescens IID618 | – | 0.10 | 0.10 | 0.10 | 0.20 | 0.05 |
| S. enteritidis IID604 | – | 0.05 | 0.05 | 0.10 | 0.10 | 0.025 |
| S. sonnei IID969 | – | 0.0125 | 0.0125 | 0.025 | 0.025 | 0.0125 |
| P. aeruginosa V-1 | – | 0.20 | 0.20 | 0.39 | 0.39 | 0.05 |
| P. aeruginosa IFO12689 | – | 0.78 | 0.78 | 1.56 | 1.56 | 0.20 |
| P. aeruginosa IID1210 | – | 0.78 | 0.78 | 1.56 | 1.56 | 0.39 |
| P. cepacia GIFU518 | – | 0.39 | 0.78 | 0.78 | 0.78 | 0.78 |
| P. maltophilia GIFU2491 | – | 0.10 | 0.10 | 0.10 | 0.20 | 0.78 |
| A. anitratus IID876 | – | 0.05 | 0.05 | 0.05 | 0.10 | 0.20 |
| A. faecalis 0114002 | – | 0.39 | 0.39 | 0.39 | 0.39 | 0.39 |
| B. fragilis GM7000 | – | 0.10 | 0.10 | 0.39 | 0.78 | 3.13 |
| B. thetaiotaomicron 0661 | – | 0.20 | 0.39 | 0.39 | 1.56 | 25 |
| C. difficile I-E | + | 0.39 | 0.39 | 0.78 | 3.13 | 12.5 |
| C. perfringens KYA13123 | + | 0.10 | 0.20 | 0.20 | 0.39 | 0.39 |

Reference compound CPFX: Ciprofloxacin

Test Example 2 - Therapeutic effect on the systemic infection of mouse

The infectious bacteria were injected intraperitoneally using five ICR mice per group. one hour later, 5 doses of drugs were administered once orally, respectively, and, from a curve of survival rate of mouse as a function of dose of drug, a dose ($ED_{50}$) by which 50% of animals escape the death to heal was determined.

Table 2 shows the results.

Table 2

Therapeutic effect on systemic infection of mouse

| Compound | $ED_{50}$ (mg/kg) | |
|---|---|---|
| | E. coli [a)] | S. aureus [b)] |
| Example 33 | 0.31 | 1.21 |
| Example 34 | 0.26 | 1.39 |
| Example 35 | 1.11 | 2.00 |
| Example 36 | 2.04 | 6.08 |
| CPFX | 0.49 | 20.3 |

a): E. coli ML 4707: Level of bacteria inoculated, $8.8 \times 10^6$ CFU/mouse

b): S. aureus Smith: Level of bacteria inoculated, $4.8 \times 10^6$ CFU/mouse

## Claims

1. An optically active compound which is an 8-methoxyquinolonecarboxylic acid represented by the formula (I)

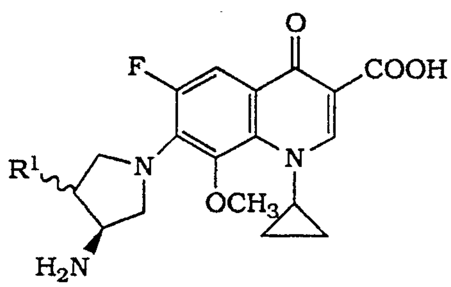

(I)

wherein $R^1$ is lower alkyl, the absolute configuration being the S- or R-configuration; a hydrate thereof or a pharmaceutically acceptable salt thereof.

2. The optically active 8-methoxyquinolonecarboxylic acid salt according to Claim 1, which is 7-(3(S)-amino-4(R)-methyl-l-pyrrolidinyl)-l-cyclopropyl-6-fluoro-1,4-dihydro-8-methoxy-4-oxo-3-quinolonecarboxylic acid hydrochloride substantially free of other optical isomers thereof.

3. The optically active 8-methoxyquinolonecarboxylic acid salt according to Claim 1, which is 7-(3(S)-amino-4(S)-methyl-l-pyrrolidinyl)l-cyclopropyl-6-fluoro-1,4-dihydro-8-methoxy-4-oxo-3-quinolonecarboxylic acid hydrochloride substantially free of other optical isomers thereof.

4. The optically active 8-methoxyquinolonecarboxylic acid salt according to Claim 1, which is 7-(3(R)-amino-4(R)-methyl-l-pyrrolidinyl)-l-cyclopropyl-6-fluoro-1,4-dihydro-8-methoxy-4-oxo-3-quinolonecarboxylic acid hydrochloride substantially free of other optical isomers thereof.

5. The optically active 8-methoxyquinolonecarboxylic acid salt according to Claim 1, which is 7-(3(R)-amino-4(S)-methyl-l-pyrrolidinyl)-l-cyclopropyl-6-fluoro-1,4-dihydro-8-methoxy-4-oxo-3-quinolonecarboxylic acid hydrochloride substantially free of other optical isomers thereof.

6. The optically active compound according to Claim 1, wherein the amino group at the 3-position of the pyrrolidine ring is in the S-configuration.

7. A process for the preparation of an optically active compound which is an 8-methoxyquinolonecarboxylic acid represented by the formula (I)

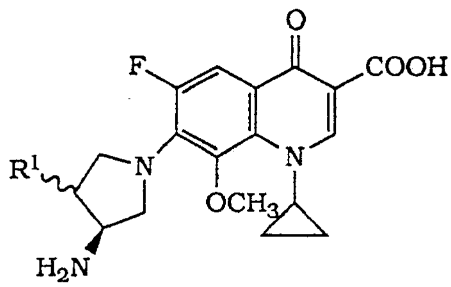

(I)

wherein $R^1$ is a lower alkyl group, the absolute configuration being the S- or R-configuration, a hydrate thereof or a pharmaceutically acceptable salt thereof, said process comprising:

condensing a compound of the formula (II)

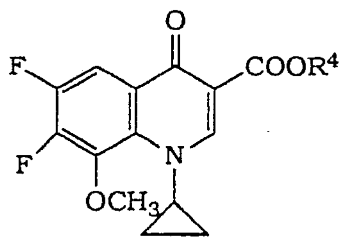

(II)

wherein $R^4$ is hydrogen or lower alkyl with a compound of the formula (III)

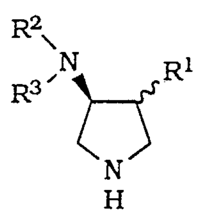

(III)

wherein $R^1$ is as defined above, and $R^2$ and $R^3$ each independently represent hydrogen or a protective group for the amino group, or $R^2$ and $R^3$ together form a protective group for the amino group; and

when $R^2$ or $R^3$ is a protective group, or when $R^2$ and $R^3$ together form a protective group, eliminating the protective groups; and

when $R^4$ is lower alkyl, hydrolyzing the carboxylic acid ester to convert $R^4$ to hydrogen.

8. An antibacterial agent comprising an optically active antibacterially effective amount of an 8-methoxyquinolonecarboxylic acid represented by the formula (I)

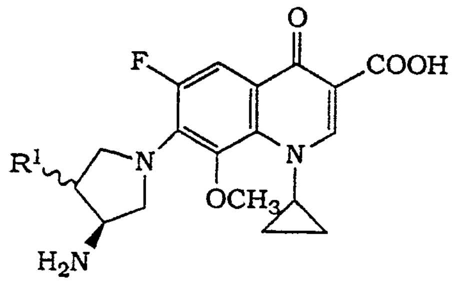

(I)

wherein $R^1$ is a lower alkyl, the absolute configuration being the S- or R-configuration, a hydrate thereof or a pharmaceutically acceptable salt thereof; and a pharmaceutically acceptable diluent.

**9.** The antibacterial agent according to Claim 8, wherein the amino group at the 3-position of the pyrrolidine ring of the 8-methoxyquinolonecarboxylic acid represented by the formula (I) is in the S-configuration.

**10.** The antibacterial agent according to Claim 9, wherein said 8-methoxyquinolonecarboxylic acid is 7-(3-(S)amino-4(R)-methyl-l-pyrrolidinyl)-l-cyclopropyl-6-fluoro-1,4dihydro-8-methoxy-4-oxo-3-quinolonecarboxylic acid.

**11.** The antibacterial agent according to Claim 9, wherein said 8-methoxyquinolonecarboxylic acid is 7-(3-(S)amino-4(S)-methyl-l-pyrrolidinyl)-l-cyclopropyl-6-fluoro-1,4dihydro-8-methoxy-4-oxo-3-quinolonecarboxylic acid.

**12.** The use of an optically active 8-methoxyquinolonecarboxylic acid represented by the formula (I)

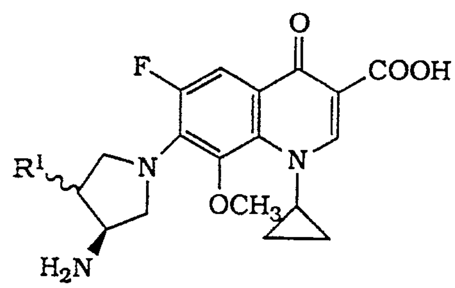

(I)

wherein $R^1$ is a loner alkyl group, the absolute configuration being the S- or R-configuration, a hydrate thereof or a pharmaceutically acceptable salt thereof, for the manufacture of a pharmaceutical composition for treating a bacterial infection in a patient.

**13.** The use according to Claim 12, wherein said bacterial infection is caused by a Gram-positive bacterium.

**14.** The use according to Claim 12, wherein the amino group at the 3-position ofthe pyrrolidine ring of the 8-methoxyquinolonecarboxylic acid represented by the formula (I) is in the S-configuration.

**15.** The use according to Claim 14, wherein said 8-methoxyquinolonecarboxylic acid is 7-(3(S)-amino-4(R)-methyl-1-pyrrolidinyl)-l-cyclopropyl-6-fluoro-1,4-dihydro-8-methoxy4-oxo-3-quinolonecarboxylic acid.

**16.** The use according to Claim 14, wherein said 8-methoxyquinolonecarboxylic acid is 7-(3(S)-amino-4(S)-methyl-1-pyrrolidinyl)-l-cyclopropyl-6-fluoro-1,4-dihydro-8-methoxy-4-oxo-3-quinolonecarboxylic acid.

**17.** An optically active pyrrolidine represented by the formula (IV)

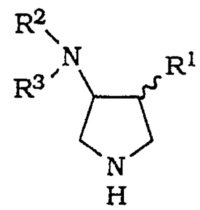

(IV)

wherein $R^1$ is lower alkyl, and $R^2$ and $R^3$ each independently represent hydrogen or a protective group for the amino group, or $R^2$ and $R^3$ together form a protective group for the amino group; wherein the substituent at the 3-position is in the S- or R-configuration and the substituent at the 4-position is in the S- or R-configuration.

**18.** The optically active pyrrolidine according to Claim 17, which is 3(S)-((t-butoxycarbonyl)amino)-4(S)-methylpyrrolidine.

**19.** The optically active pyrrolidine according to Claim 17, which is 3(S)-((t-butoxycarbonyl)amino)-4(R)-methylpyrrolidine.

**20.** An optically active pyrrolidine represented by the formula (V)

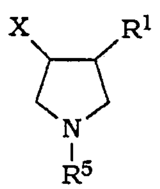

(V)

wherein $R^5$ is benzyl, diphenylmethyl, trityl, p-methoxybenzyl, p-methoxyphenyl or l-phenethyl,
$R^1$ is lower alkyl, and
X is amino, protected amino, hydroxyl, protected hydroxyl, p-toluenesulfonyloxy, methanesulfonyloxy, azido or halogen; and wherein the substituent at the 3-position is in the S- or R-configuration and the substituent at the 4-position is in the S- or R-configuration.

**21.** An optically active butane represented by the formula (VI)

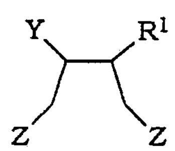

(VI)

wherein Y is protected amino, hydroxyl, protected hydroxyl or halogen,
Z is hydroxyl, p-toluenesulfonyl-oxy, methanesulfonyloxy or halogen, and
$R^1$ is lower alkyl; and
wherein the substituent at the 2-position is in the S- or R-configuration and the substituent at the 3-position is in the S- or R-configuration.

**22.** A process for the preparation of optically active compounds of the formula (IV)

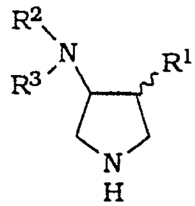

(IV)

wherein $R^1$ is lower alkyl,

$R^2$ and $R^3$ each independently represent hydrogen or a protective group for the amino group, or $R^2$ and $R^3$ together form a protective group for the amino group, wherein the substituent at the 3-position of the pyrrolidine ring is in the S- or R-configuration and the substituent at the 4-position of the pyrrolidine ring is in the S- or R-configuration, said process comprising:

converting a compound of the formula (VII)

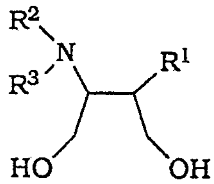

(VII)

wherein $R^1$, $R^2$ and $R^3$ are as defined above, and wherein the substituent at the 2-position of the butane is in the S- or R- configuration and the substituent at the 3-position of the butane is in the S- or R-configuration, to a compound of the formula (VII')

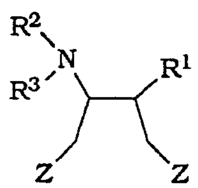

(VII')

wherein Z is p-toluenesulfonyloxy, methanesulfonyloxy or halogen, and $R^1$, $R^2$ and $R^3$ are as defined above;

reacting the compound of formula (VII') with a primary amine of the formula (VIII)

$R^5$-$NH_2$ (VIII)

to form a compound of the formula (IX)

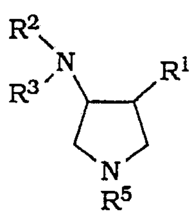

(IX)

wherein $R^5$ is benzyl, diphenylmethyl, trityl, p-methoxybenzyl, p-methoxyphenyl or l-phenethyl,

$R^1$, $R^2$ and $R^3$ are as defined above, and wherein the substituent at the 3-position of the pyrrolidine ring is in the S- or R-configuration and the substituent at the 4-position of the pyrrolidine ring is in the S- or R-configuration; and

then, removing the protective group $R^5$ from the ring nitrogen atom to produce the compound of the formula (IV).

**23.** A process for the preparation of optically active compounds of the formula (IV)

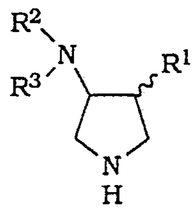

(IV)

wherein $R^1$ is lower alkyl, $R^2$ and $R^3$ each independently represent hydrogen or a protective group for the amino group, or $R^2$ and $R^3$ together form a protective group for the amino group, wherein the substituent at the 3-position is in the S- or R-configuration and the substituent at the 4-position is in the S- or R-configuration, said process comprising:

converting a compound of the formula (X)

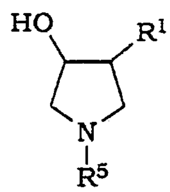

(X)

wherein $R^1$ is as defined above and $R^5$ is benzyl, diphenylmethyl, trityl, p-methoxybenzyl, p-methoxyphenyl or 1-phenethyl, to a compound of the formula (X')

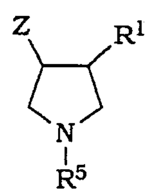

(X')

wherein $R^1$ and $R^5$ are as defined above, and Z is p- toluenesulfonyloxy, methanesulfonyloxy or halogen;

reacting the compound of the formula (X') with a metal azide or quaternary ammonium azide to obtain sterically reversed compounds of the formula (XI)

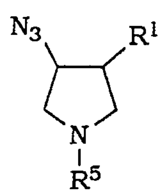

(XI)

wherein $R^1$ and $R^5$ are as defined above;

reducing the compound ofthe formula (XI) to obtain compounds of the formula (XII)

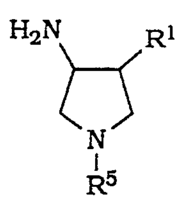

(XII)

wherein $R^1$ and $R^5$ are as defined above;

introducing protective groups for the amino group of the compounds of formula (XII) to form compounds of the formula (IX)

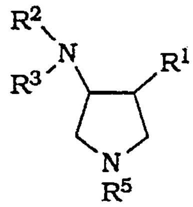

(IX)

wherein $R^1$ and $R^5$ are as defined above, and wherein $R^2$ and $R^3$ each independently represent a protective group for the amino group or $R^2$ and $R^3$ together form a protective group for the amino group; and

then removing the protective group $R^5$ from the ring nitrogen atom to produce the compound of the formula (IV).

24. The process according to Claim 23, wherein said removal of the protective group $R^5$ from the ring nitrogen atom is effected through reduction or oxidation.

**Claims for the following Contracting State: ES**

1. A process for the preparation of an cptically active compound which is an 8-methoxyquinolonecarboxylic acid represented by the formula (I)

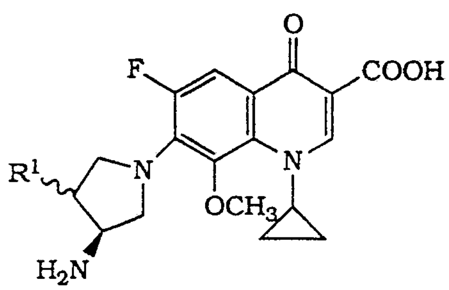

(I)

wherein $R^1$ is a lower alkyl group, the absolute configuration being the S- or R-configuration, a hydrate thereof or a pharmaceutically acceptable salt thereof, said process comprising:

condensing a compound of the formula (II)

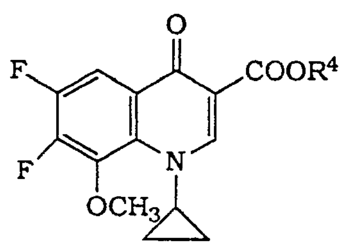

(II)

wherein $R^4$ is hydrogen or lower alkyl with a compound of the formula (III)

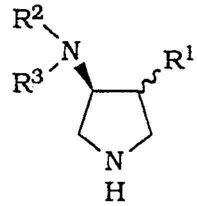

(III)

wherein $R^1$ is as defined above, and $R^2$ and $R^3$ each independently represent hydrogen or a protective group for the amino group, or $R^2$ and $R^3$ together form a protective group for the amino group; and

when $R^2$ or $R^3$ is a protective group, or when $R^2$ and $R^3$ together form a protective group, eliminating the protective groups; and

when $R^4$ is lower alkyl, hydrolyzing the carboxylic acid ester to convert $R^4$ to hydrogen.

2. The process according to Claim 1 for the manufacture of 7-(3(5) -amino-4(R)-methyl-l-pyrrolidinyl)-l-cyclopropyl-6-fluoro-1,4-dihydro-8-methoxy-4-oxo-3-quinolonecarboxylic acid hydrochloride substantially free of other optical isomers thereof.

3. The process according to Claim 1 for the manufacture of 7-(3(S)-amino-4(S)-methyl-l-pyrrolidinyl)-l-cyclopropyl-6-fluoro-1,4-dihydro-8-methoxy-4-oxo-3-quinolonecarboxylic acid hydrochloride substantially free of other optical isomers thereof.

4. The process according to Claim 1 for the manufacture of 7-(3(R)-amino-4(R)-methyl-l-pyrrolidinyl)-l-cyclopropyl-6-fluoro-1,4-dihydro-8-methoxy-4-oxo-3-quinolonecarboxylic acid hydrochloride substantially free of other optical isomers thereof.

5. The process according to Claim 1 for the manufacture of 7-(3(R)-amino-4(S)-methyl-l-pyrrolidinyl)-l-cyclopropyl-6-fluoro-1,4-dihydro-8-methoxy-4-oxo-3-quinolonecarboxylic acid hydrochloride substantially free of other optical isomers thereof.

6. The process according to Claim 1 for the manufacture of the optically active compound according to Claim 1, wherein the amino group at the 3-position of the pyrrolidine ring is in the S-configuration.

7. The use of an optically active 8-methoxyquinolonecarboxylic acid represented by the formula (I)

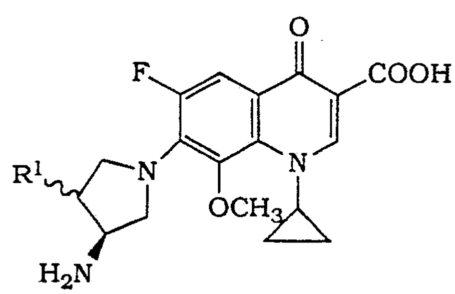

(I)

wherein $R^1$ is a lower alkyl group, the absolute configuration being the S- or R-configuration, a hydrate thereof or a pharmaceutically acceptable salt thereof, for the manufacture of a pharmaceutical composition for treating a bacterial infection in a patient.

8. The use according to Claim 7, wherein said bacterial infection is caused by a Gram-positive bacterium.

9. The use according to Claim 7, wherein the amino group at the 3-position ofthe pyrrolidine ring ofthe 8-methoxyquinolonecarboxylic acid represented by the formula (I) is in the S-configuration.

10. The use according to Claim 9, wherein said 8-methoxyquinolonecarboxylic acid is 7-(3(S)-amino-4(R)-methyl -l -pyrrolidinyl)-l-cyclopropyl-6fluoro-1,4-dihydro-8-methoxy4-oxo-3-quinolonecarboxylic acid.

11. The use according to Claim 9, wherein said 8-methoxyquinolonecarboxylic acid is 7-(3(S)-amino-4(S)-methyl-1-pyrrolidinyl)-l-cyclopropyl-6-fluoro-1,4-dihydro-8-methoxy-4-oxo-3-quinolonecarboxylic acid.

12. A process for the preparation of optically active compounds of the formula (IV)

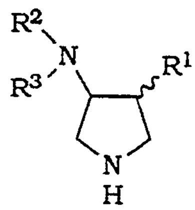

(IV)

wherein $R^1$ is lower alkyl,

$R^2$ and $R^3$ each independently represent hydrogen or a protective group for the amino group, or $R^2$ and $R^3$ together form a protective group for the amino group, wherein the substituent at the 3-position of the pyrrolidine ring is in the S- or R-configuration and the substituent at the 4-position of the pyrrolidine ring is in the S- or R-configuration, said process comprising:

converting a compound of the formula (VII)

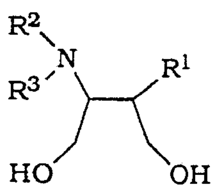

(VII)

wherein $R^1$, $R^2$ and $R^3$ are as defined above, and wherein the substituent at the 2-position of the butane is in the S- or R- configuration and the substituent at the 3-position of the butane is in the S- or R-configuration, to a compound of the formula (VII')

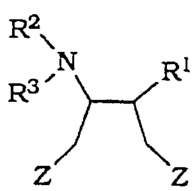

(VII')

wherein Z is p-toluenesulfonyloxy, methanesulfonyloxy or halogen, and $R^1$, $R^2$ and $R^3$ are as defined above;

reacting the compound of formula (VII') with a primary amine of the formula (VIII)

$R^5$-$NH_2$ (VIII)

to form a compound of the formula (IX)

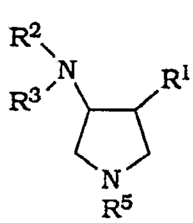

(IX)

wherein $R^5$ is benzyl, diphenylmethyl, trityl, p-methoxybenzyl, p-methoxyphenyl or l-phenethyl,

$R^1$, $R^2$ and $R^3$ are as defined above, and wherein the substituent at the 3-position of the pyrrolidine ring is in the S- or R-configuration and the substituent at the 4-position of the pyrrolidine ring is in the S- or R-configuration; and

then, removing the protective group $R^5$ from the ring nitrogen atom to produce the compound of the formula (IV).

**13.** A process for the preparation of optically active compounds of the formula (IV)

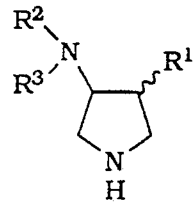

(IV)

wherein $R^1$ is lower alkyl,

$R^2$ and $R^3$ each independently represent hydrogen or a protective group for the amino group, or $R^2$ and $R^3$ together form a protective group for the amino group, wherein the substituent at the 3-position is in the S- or R-configuration and the substituent at the 4-position is in the S- or R-configuration, said process comprising:

converting a compound of the formula (X)

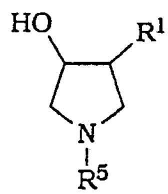

(X)

wherein $R^1$ is as defined above and $R^5$ is benzyl, diphenylmethyl, trityl, p-methoxybenzyl, p-methoxyphenyl or 1-phenethyl, to a compound of the formula (X')

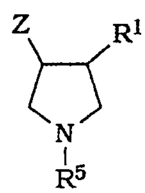

(X')

wherein $R^1$ and $R^5$ are as defined above, and Z is p- toluenesulfonyloxy, methanesulfonyloxy or halogen;

reacting the compound of the formula (X') with a metal azide or quaternary ammonium azide to obtain sterically reversed compounds of the formula (XI)

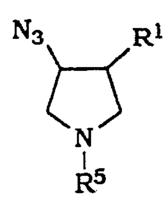

(XI)

wherein $R^1$ and $R^5$ are as defined above;

reducing the compound of the formula (XI) to obtain compounds of the formula (XII)

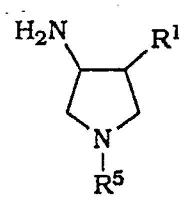

(XII)

wherein $R^1$ and $R^5$ are as defined above:

Introducing protective groups for the amino group of the compounds of formula (XII) to form compounds of the formula (IX)

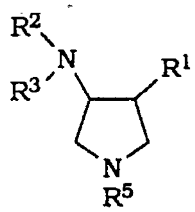

(IX)

wherein $R^1$ and $R^5$ are as defined above, and wherein $R^2$ and $R^3$ each independently represent a protective group for the amino group or $R^2$ and $R^3$ together form a protective group for the amino group; and

then removing the protective group $R^5$ from the ring nitrogen atom to produce the compound of the formula (IV).

**14.** The process according to Claim 23, wherein said removal of the protective group $R^5$ from the ring nitrogen atom is effected through reduction or oxidation.

European Patent Office

# EUROPEAN SEARCH REPORT

Application Number

**EP 91 10 2291**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | EP-A-0 230 295 (KYORIN PHARMACEUTICAL CO., LTD.) * claims 1 - 3; examples 5, 6; abstract & JP-A-62-252772 * | 1,7,8,12 | C 07 D 401/04<br>C 07 D 207/10<br>C 07 D 207/12<br>C 07 D 207/14<br>C 07 D 309/12<br>C 07 C 31/34<br>C 07 C 271/16<br>C 07 C 309/42<br>A 61 K 31/47 |
| Y | EP-A-0 302 371 (ABBOTT LABORATORIES) * page 2, line 12 - page 3, line 34 * * page 6, lines 27 - 58 * | 1,7,8,12 | |
| Y | EP-A-0 347 851 (SHIONOGI SEIYAKU K.K.) * page 4, lines 24 - 30 * * page 18, lines 10 - 20 * * examples 1 - 4 * | 1,7,8,12 | |
| X,A | EP-A-0 341 493 (DAIICHI SEIYAKU CO. LTD.) * pages 34, 35, reference examples 33-3, 33-4, and 33-5 * * page 42, compounds 25, 26; page 45, compounds 55b, 56b; claims 1, 9 * * examples 1 - 4, 10 - 13 * | 17,20,1,7, 8 | |
| A | JOURNAL OF MEDICINAL CHEMISTRY, vol. 14, no. 1, 1971, pages 24 - 30; D.T. WITIAK et al.: "L[S]- and D[R]-3-Amino-1-phenylpyrrolidines. Stereoselective Antagonists for Histamine and Acetylcholine Receptors in Vitro" * page 25,compound 1; page 29,last paragraph to page 30,first paragraph | 17 | **TECHNICAL FIELDS SEARCHED (Int. Cl.5)** |
| X | TETRAHEDRON LETTERS, vol. 30, no. 48, 1989, pages 6721 - 6724; H. TOSHIMA et al.: "Synthetic study on aplysiatoxins, highly inflammatory agents and tumor promoters,Synthesis of the three optically active segments" * page 6723, compound 14 * | 21 | C 07 C 31/00<br>C 07 C 271/00<br>C 07 C 309/00<br>C 07 D 207/00<br>C 07 D 215/00<br>C 07 D 309/00<br>C 07 D 401/00 |
| A | PATENT ABSTRACTS OF JAPAN vol. 9, no. 247 (C-307)(1970) 03 October 1985, & JP-A-60 104061 (SANKYO K.K.) 08 June 1985, * the whole document * | 21,22 | |

-/-

The present search report has been drawn up for all claims

| Place of search | Date of completion of search | Examiner |
|---|---|---|
| Berlin | 03 June 91 | HASS C V F |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document

European Patent Office

# EUROPEAN SEARCH REPORT

Application Number

EP 91 10 2291

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | GB-A-2 013 662 (SOCIETE D'ETUDES SCIENTIFIQUES ET INDUSTRIELLES D'ILE DE FRANCE)<br>* page 2, compound (IV) and "diagram of reaction" *<br>– – – | 21,22 | |
| A | EP-A-0 218 249 (TOKYO KASEI KOGYO CO., LTD.)<br>* claim 5 *<br>– – – – – | 22 | |
| | | | TECHNICAL FIELDS SEARCHED (Int. Cl.5) |

The present search report has been drawn up for all claims

| Place of search | Date of completion of search | Examiner |
|---|---|---|
| Berlin | 03 June 91 | HASS C V F |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

& : member of the same patent family, corresponding document